(19) Europäisches Patentamt
European Patent Office
Office européen des brevets

(11) **EP 3 747 911 A1**

(12) **EUROPEAN PATENT APPLICATION**
published in accordance with Art. 153(4) EPC

(43) Date of publication:
**09.12.2020 Bulletin 2020/50**

(51) Int Cl.:
*C08B 15/00* [(2006.01)]      *A61K 8/73* [(2006.01)]
*A61Q 1/00* [(2006.01)]      *A61Q 19/00* [(2006.01)]

(21) Application number: **19747667.4**

(22) Date of filing: **01.02.2019**

(86) International application number:
**PCT/JP2019/003646**

(87) International publication number:
**WO 2019/151486 (08.08.2019 Gazette 2019/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.02.2018 JP 2018016310**

(71) Applicant: **Nissan Chemical Corporation
Tokyo 103-6119 (JP)**

(72) Inventor: **IMOTO Takayuki
Funabashi-shi, Chiba 274-0052 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **FUNCTIONAL POLYSACCHARIDE PARTICLE**

(57)    An object is to provide a cellulose particle which is a natural material and soft, and excellent in light scattering properties.

This is to provide a particle containing cellulose or a cellulose derivative as a main component, and having a wrinkle-like or fold-like uneven structure on a surface, and a method for producing the same. Such a particle is soft and excellent in light scattering properties, so that it is useful as cosmetics.

Fig. 3

**Description**

TECHNICAL FIELD

The present invention relates to a particle containing cellulose or a cellulose derivative as a main component, which comprises having a wrinkle-like or fold-like uneven structure on a surface thereof, a method for producing the same, and cosmetics containing said particles.

BACKGROUND ART

Porous fine particles have been used in a wide range of fields as various kinds of additives such as abrasives, matting agents (light diffusing agents), carriers, water absorbing materials, fillers, anti-blocking agents, etc., depending on the material, shape, physical properties, etc.

Among porous fine particles, microbeads generally refer to fine particles (particles having several μm to several hundred μm) made of plastics such as polyethylene, polypropylene, etc., and have widely been used for perfumery such as toiletry products (for example, face wash, body wash), oral care products (for example, dentifrices), cosmetics (for example, foundation), etc. Specifically, microbeads have been used as a scrubbing agent to enhance a massage effect and a cleaning effect in toiletry products such as face wash, body wash, etc., and in cosmetics such as foundation, etc., for example, they are used for developing a defocus effect (also referred to as a soft focus effect) that scatters reflected light in multiple directions, and corrects defects of skins such as spots, wrinkles, freckles, color unevenness, and the like (for example, see Patent Documents 1 and 2).

However, in recent years, it has been feared that microbeads pass through sewage treatment and flow out into rivers and oceans, and are taken up by living organisms to adversely affect the ecosystem. Since it is almost impossible to collect the microbeads once they flow into the environment, it has been required to replace the microbeads made of plastics with an environment-friendly material such as biodegradable plastic, a natural material, etc. As such a material, for example, porous resin fine particles containing a polyester-based thermoplastic resin having biodegradability as a main component have been reported (for example, see Patent Document 3). Also, as microbeads derived from natural materials, cellulose particles have been known (for example, see Patent Document 4).

PRIOR ART DOCUMENTS

PATENT DOCUMENTS

    Patent Document 1: JP 2016-40264A
    Patent Document 2: WO 2010/092890
    Patent Document 3: WO 2017/056908
    Patent Document 4: JP Hei.9-132601A

SUMMARY OF THE INVENTION

PROBLEMS TO BE SOLVED BY THE INVENTION

However, since existing cellulose particles are hard and inferior in light scattering property, further improvement of these properties has been demanded for use in perfumery. That is, an object of the present invention is to provide a cellulose particle which is a natural material and is soft, and has excellent light scattering properties.

MEANS TO SOLVE THE PROBLEMS

As a result of intensive studies by the present inventors, they have found that particles obtained by spray-drying a dispersion liquid of cellulose or a cellulose derivative, in particular, plant-derived cellulose nanofiber is appropriately suppressed in crystallization degree, and has a wrinkle-like or fold-like uneven structure on a surface thereof so that it is soft and excellent in light scattering properties and oil absorption properties, whereby the present invention has been completed. The present invention is as follows.

    (1) A particle containing cellulose or a cellulose derivative as a main component, which comprises having a wrinkle-like or fold-like uneven structure on a surface and a crystallization degree of 80% or less.

(2) A particle which comprises having a light scattering rate in a range of 50 to 230%, and containing cellulose or a cellulose derivative as a main component.

(3) The particle described in the above-mentioned (1), wherein a light scattering rate is in a range of 50 to 230%.

(4) The particle described in the above-mentioned (2), wherein a crystallization degree is 80% or less.

(5) The particle described in the above-mentioned (1) to (4), wherein the cellulose is plant-derived cellulose, cellulose fiber or cellulose nanofiber.

(6) A method for producing a particle containing cellulose or a cellulose derivative as a main component, having a wrinkle-like or fold-like uneven structure on a surface and a crystallization degree of 80% or less, which comprises a step of obtaining a dispersion liquid of cellulose or a cellulose derivative, and a step of spray-drying the obtained dispersion liquid.

(7) A method for producing a particle containing cellulose or a cellulose derivative as a main component and having a light scattering rate in a range of 50 to 230% , which comprises a step of obtaining a dispersion liquid, and a step of spray-drying the obtained dispersion liquid.

(8) The producing method described in (6), wherein a light scattering rate of the particles containing the cellulose or the cellulose derivative as a main component is in a range of 50 to 230%.

(9) The producing method described in the above-mentioned (7), wherein a crystallization degree of the particles containing the cellulose or the cellulose derivative as a main component is 80% or less.

(10) The producing method described in the above-mentioned (6) to (9), wherein the cellulose is plant-derived cellulose, cellulose fiber or cellulose nanofiber.

(11) The producing method described in any of the above-mentioned (6) to (10), wherein a concentration of the cellulose or the cellulose derivative in the dispersion liquid is 0.5 to 5% by mass.

(12) The producing method described in any of the above-mentioned (6) to (11), wherein the dispersion liquid is obtained by physical pulverization of the cellulose or the cellulose derivative.

(13) A cosmetic which comprises the particle described in any of the above-mentioned (1) to (5), or the particle obtained by the producing method described in any of the above-mentioned (6) to (12).

EFFECTS OF THE INVENTION

The particle of the present invention is appropriately suppressed in the crystallization degree, and has a wrinkle-like or fold-like uneven structure on the surface thereof, so that it is soft and is suitable for adding to cosmetics that comes into direct contact with skin. In particular, the particle of the present invention is excellent in oil absorption properties, so that it is suitable for adding to cosmetics containing an oily component(s) and an oily base(s), and it has also excellent optical characteristics (light scattering properties) that the incident light is uniformly light scattered, so that it can be expected to exhibit a defocus effect (also called a soft focus effect) by formulating it into cosmetics such as a foundation, etc.

BRIEF DESCRIPTION OF THE DRAWINGS

FIG. 1 is a graph showing the correlation between the incident light angle and the light scattering rate of the particles obtained in Examples 1 to 3 and the particles of Comparative Examples 1 and 2.

FIG. 2(a) is a graph showing the reflected light intensity of the particles obtained in Example 1 by measuring distribution of the reflected light at the incident light -45°, and (b) is a graph showing the reflected light intensity of the cellulose particles of Comparative Example 1 (control) by measuring distribution of the reflected light at the incident light -45°.

FIG. 3(a) is a scanning electron microscope (SEM) photograph of the particles obtained in Example 1, (b) is an SEM photograph of the particles obtained in Example 2, (c) is an SEM photograph of the particles obtained in Example 3, (d) is an SEM photograph of the particles obtained in Example 4, and (e) is an SEM photograph of the particles obtained in Example 5.

FIG. 4 is a transmission electron microscope (TEM) photograph of a cross section of the particles obtained in Example 1.

FIG. 5 is a graph showing the results of evaluation of the particles obtained in Example 1, and commercially available cellulose particles and Nylon particles as controls by fluctuation (MMD) of an average friction coefficient obtained by a friction tester.

FIG. 6 is a graph showing the results of evaluation of the particles obtained in Example 1 and commercially available cellulose particles and Nylon particles as controls by an average friction coefficient (MIU) obtained by a friction tester.

FIG. 7(a) is a powder X-ray diffraction diagram of the particles obtained in Example 1, (b) is a powder X-ray diffraction diagram of the particles obtained in Example 3, and (c) is a powder X-ray diffraction diagram of the commercially available cellulose particles of Comparative Example 1.

FIG. 8(a) is a volume particle size distribution and passage amount integration of the particles obtained in Example 1, (b) is a volume particle size distribution and passage amount integration of the particles obtained in Example 2, (c) is a volume particle size distribution and passage amount integration of the particles obtained in Example 3, (d) is a volume particle size distribution and passage amount integration of the particles obtained in Example 4, and (e) is a volume particle size distribution and passage amount integration of the particles obtained in Example 5.

## EMBODIMENTS TO CARRY OUT THE INVENTION

<Particle>

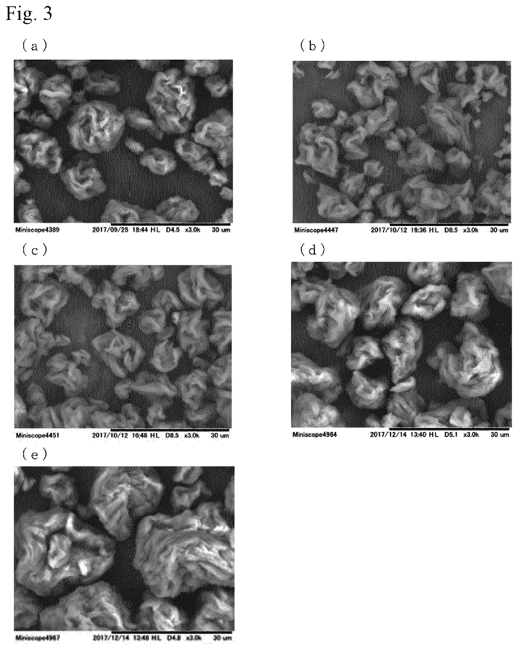

The present invention relates to a particle which comprises having a wrinkle-like or fold-like uneven structure on a surface, and containing cellulose or a cellulose derivative as a main component. "Having a wrinkle-like or fold-like uneven structure" means that, as shown in FIG. 3, when an enlarged image of a particle is observed, the surface thereof is not smooth and has groove-shaped lines which have a wrinkle-like or fold-like appearance. This is different from the hemispherical crater-like depressions found in other porous spherical cellulose particles, and exhibits an irregular uneven structure found in petal-shaped particles.

(Cellulose)

The particles of the present invention contains cellulose or a cellulose derivative as a main component. The cellulose or the cellulose derivative used in the present invention may be mentioned natural fibers such as wool, cotton, silk, hemp, pulp, etc., those derived from regenerated fibers such as rayon, polynosic, cupra (Bemberg (Registered Trademark)), lyocell (Tencel (Registered Trademark)), etc., or cellulose produced by bacteria. In addition, it may be derived from cellulose composite fibers of cellulose fibers and synthetic fibers (for example, polyolefin fibers such as polyethylene, polypropylene, etc.).

As the cellulose or the cellulose derivative to be used in the present invention, there may be mentioned those derived from natural fibers, for example, those derived from plants such as wood, bamboo, hemp, jute, kenaf, cotton, beet, agricultural waste products, etc., in particular, there may be mentioned those derived from broadleaf tree, acicular tree or bamboo. Also, it may be a material in which $\alpha$-cellulose obtained from such a fibrous plant may be partially depolymerized with an acid and purified, for example, crystalline cellulose.

Also, in the present invention, it is preferable to use cellulose fibers, in particular, cellulose nanofibers as the cellulose or the cellulose derivative. The "cellulose nanofiber (CNF)" is a fiber obtained by defibrating cellulose fibers to a nanosize level, and is generally a fiber having a fiber width of about 4 to 200 nm and a fiber length of about 5 $\mu$m or more. Such cellulose nanofibers can be prepared by a known method and can be obtained as a commercially available product. For example, it can be obtained from suppliers such as Daio Paper Corporation and Chuetsu Pulp & Paper Co., Ltd., etc.

In the present invention, "containing cellulose or a cellulose derivative as a main component" means that the ratio (weight basis) of the cellulose or the cellulose derivative occupied in the particles is more than 50% by mass. The ratio (weight basis) of the cellulose or the cellulose derivative is preferably 60% by mass or more, more preferably 70% by mass or more, further preferably 80% by mass or more, and particularly preferably 90% by mass or more. In the most preferable embodiment, the particles of the present invention consists of the cellulose or the cellulose derivative.

As the component other than the cellulose or the cellulose derivative contained in the particles, there may be mentioned inorganic pigments or clay minerals and, for example, mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate metal salt, magnesium, zeolite, barium sulfate, calcined calcium sulfate, calcium phosphate, fluoroapatite, hydroxyapatite, ceramic powder, bentonite, smectite, clay, mud, metallic soap (for example, zinc myristate, calcium palmitate, aluminum stearate), calcium carbonate, red iron oxide, yellow iron oxide, black iron oxide, ultramarine, Prussian blue, carbon black, titanium oxide, fine particle and ultrafine particle titanium oxide, zinc oxide, fine particle and ultrafine particle zinc oxide, alumina, silica, fumed silica (ultrafine particle silicic anhydride), titanated mica, fish scale, boron nitride, photochromic pigments, synthetic fluorophlogopite, fine particle complex powders, inorganic powders with various kinds of sizes and shapes such as gold, aluminum, etc., and a powder, etc. in which these are hydrophobized or hydrophilized by treatment with various kinds of surface treatment agent such as silicone including hydrogen silicone, cyclic hydrogen silicone, etc., or other silanes or a titanium coupling agent, can be used.

A particle diameter of the particles of the present invention can be appropriately set depending on the desired use of the particles and, for example, it is distributed in the range of 0.5 to 500 µm, preferably in the range of 1 to 200 µm, more preferably 2 to 100 µm, and particularly preferably in the range of 5 to 80 µm, and an average particle diameter is, for example, in the range of 5 to 40 µm, and preferably in the range of 5 to 30 µm. Incidentally, in the present invention, the particle size means a value measured by a scattering type particle diameter distribution measurement device, and an average particles diameter means an arithmetic mean diameter calculated from the obtained particle size distribution.

(Light scattering rate)

In another aspect, the particles of the present invention are characterized in that a light scattering rate represented by the following formula (1) is in the range of 50 to 200%.

$$\text{Light scattering rate}(\%) = \frac{(\text{Reflection intensity at angle of } 20°/\cos\ 20°\ ) + (\text{Reflection intensity at angle of } 70°/\cos\ 70°\ )}{2\times\ (\text{Reflection intensity at angle of } 5°/\cos\ 5°\ )} \times 100$$

$$\cdots\ (1)$$

{in the formula (1), the reflection intensities at angles of 20°, 70° and 5° mean intensities of the reflected light at angles of 20°, 70° and 5° of a light receiver when light is incident from a certain angle after setting the sensitivity of the light receiver is set to an arbitrary value (which is referred to as a sensitivity adjustment value) when light is incident on particles at an angle of -30° with the normal direction to the particle being 0°.}

Here, the light scattering rate is calculated in accordance with the above-mentioned formula (1) described in WO 2010/092890. For example, when the light scattering rate at the incident angle of light is -30° is to be calculated, first, the normal direction of the surface against which the sample is pressed is 0°, and after the sensitivity of the light receiver at the time of incident of light to the sample from an angle of -30° is set to be an arbitrary value (which is referred to as a sensitivity adjustment value), the intensities of the reflected light at the angles of 20°, 70° and 5° of the light receiver are measured. Then, while maintaining the initial sensitivity adjustment value, the intensities of the reflected light at the angles of 20°, 70° and 5° of the light receiver at the time of incident of light to the same sample from an angle of -45° are measured. In the case of an incident angle of -60°, a relative intensity is similarly measured, and the light scattering rate is finally calculated.

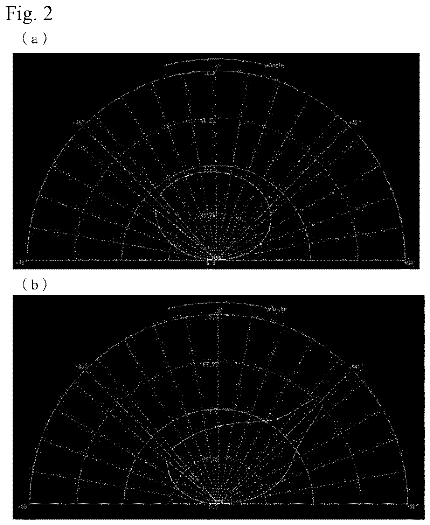

When the light scattering rate represented by the above-mentioned formula (1) becomes 100%, the graph showing reflection intensity shown in the FIG. 2 becomes circular, which means that the incident light is uniformly diffused. If this light scattering rate exceeds 100%, it means that the graph showing the reflection intensity shown in FIG. 2 becomes a horizontally long ellipse, and if it is less than 100, it means a vertically long ellipse.

The particles of the present invention has a reflected light intensity having higher uniformity despite of having a wrinkle-like or fold-like uneven structure on the surface thereof. The particles of the present invention are characterized in that the light scattering rate is in the range of 50 to 230%. At each observation of light incident angles of -30°, -45° and -60°, preferably the light scattering rate is in the range of 50 to 230% in either one of the incident angles, more preferably the light scattering rate is in the range of 50 to 230% in arbitrary two incident angles, and further preferably the light scattering rate is in the range of 50 to 230% in all of the incident angles. The light scattering rate of the particles of the present invention is preferably in the range of 50 to 200%, more preferably in the range of 70 to 190%, and further preferably in the range of 75 to 190%. This means that the particles of the present invention can enable more uniform omnidirectional reflection and, for example, when the particles of the present invention are used as an additive for cosmetics, it can be expected to develop a defocus effect.

In particular, in a measurement of distribution of the reflected light, it is preferable that a ratio of the maximum intensity of the reflected light in the vicinity of specular reflection (for example, within an angle of specular reflection ± 10°, preferably within an angle of specular reflection ± 5°) to the incident light, to the maximum intensity of the reflected light in the vicinity of the incident light (for example, within an angle of incident light ± 10°, preferably within an angle of incident light ± 5°) is in the range of 0.5 : 1 to 2 : 1, and preferably in the range of 0.5 : 1 to 1.8 : 1. When the intensity of specular reflection is in such a range, it can be expected to exhibit excellent optical characteristics, in particular, the defocus effect when the particles of the present invention are used as an additive for cosmetics (in particular, an additive for a foundation).

(Crystallization degree)

In another aspect, the particles of the present invention are characterized in that a crystallization degree is 80% or less. The crystallization degree of the present invention means a ratio of the crystalline region of the cellulose in the particles, and as described in Evaluation Example 6 mentioned later, it can be calculated from the powder X-ray diffraction patterns using the following equations (2) and (3) (see SEN-I GAKKAISHI, Vol. 46, No. 8 (1990), p.324).

$$\text{Crystallization degree of cellulose type I crystal form (\%)} = \{(I_{22.6} - I_{22.6, blank}) - (I_{18.5} - I_{18.5, blank})\} / (I_{22.6} - I_{22.6, blank}) \times 100 \quad \cdots \quad (2)$$

(wherein $I_{22.6}$ and $I_{18.5}$ indicate diffraction intensities at diffraction angles $2\theta$ of 22.6° and 18.5°, and $I_{22.6, blank}$ and $I_{18.5, blank}$ each indicate diffraction intensities of only the glass capillaries at diffraction angles $2\theta$ of 22.6° and 18.5°.)

$$\text{Crystallization degree of cellulose type II crystal form (\%)} = \{(I_{20.0} - I_{20.0, blank}) - (I_{15.0} - I_{15.0, blank})\} / (I_{20.0} - I_{20.0, blank}) \times 100 \quad \cdots \quad (3)$$

(wherein $I_{20.0}$ and $I_{15.0}$ indicate diffraction intensities at diffraction angles $2\theta$ of 20.0° and 15.0°, and $I_{20.0, blank}$ and $I_{15.0, blank}$ each indicate diffraction intensities of only the glass capillaries at diffraction angles $2\theta$ of 20.0° and 15.0°.)

When the crystallization degree of the cellulose particles is high, strength is generally improved but flexibility is lowered, so that the crystallization degree is desirably suppressed appropriately. Accordingly, the crystallization degree of the particles of the present invention is 80% or less, preferably 75% or less, and more preferably 70% or less. On the other hand, the crystallization degree of the particles of the present invention is 20% or more, preferably 25% or more, and more preferably 30% or more. In the particles of the present invention, the crystallization degree is appropriately suppressed and they have a wrinkle-like or fold-like uneven structure on the surface thereof so that they are soft and suitable for adding to cosmetics that come into direct contact with the skin.

(Oil absorption amount)

In another aspect, the particles of the present invention are characterized in that an oil absorption amount measured in accordance with JIS K 5101-13-1 is in the range of 60 to 150 g/100 g. Specifically, the particles of the present invention are placed on a measurement plate, a few drops of refined linseed oil are added thereto per a time from a spot, and each time, an operation of mixing and kneading the refined linseed oil and the particles with a spatula is repeatedly carried out, and dropping is continued until agglomerate of the refined linseed oil and the particles is formed, then, dropping one drop at a time until the agglomerate is completely mixed and kneaded which is repeated until the paste reached a smooth hardness as an end point, whereby the oil absorption amount is calculated from the refined linseed oil (g) to the measurement sample (g). The oil absorption amount of the particles of the present invention is in the range of 60 to 150 g/100 g, preferably in the range of 60 to 100 g/100 g, which are suitable for adding to cosmetics containing an oily component(s) and an oily base(s).

<Method for producing particles>

The particles of the present invention can be produced by a method which comprises a step of obtaining a dispersion liquid of cellulose or a cellulose derivative, and a step of spray-drying the obtained dispersion liquid.

In the dispersion liquid according to the producing method of the present invention, examples of cellulose or a cellulose derivative and preferred embodiments are as mentioned above. The dispersion liquid can be obtained by mixing cellulose or a cellulose derivative and a dispersing medium, and subjecting the mixture to a pulverization treatment. The dispersing medium is preferably an aqueous medium, and more preferably water, a water miscible organic solvent or a mixture thereof. Examples of the water miscible organic solvent may be mentioned an alcohol having 1 to 4 carbon atoms such as methanol, ethanol, isopropyl alcohol, butanol, etc., a ketone such as acetone, etc., a nitrile such as acetonitrile, etc., an amide such as N-methylpyrrolidone, N-cyclohexylpyrrolidone, N,N-dimethylacetamide, N,N-dimethylformamide, etc., a lactone such as $\gamma$-butyrolactone, etc., and an ether such as tetrahydrofuran, etc. In a most preferable embodiment, the dispersing medium is water, or a mixture of water and an alcohol having 1 to 4 carbon atoms.

A concentration of cellulose or a cellulose derivative in the dispersion liquid is not particularly limited as long as it is in the range capable of being used in the subsequent spray-drying step and, for example, 0.01 to 10% by mass, preferably 0.3 to 7% by mass, and more preferably 0.5 to 5% by mass.

An operation of obtaining the dispersion liquid is not particularly limited, and can be carried out using the operation for obtaining the dispersion liquid known to those skilled in the art. Typically, the dispersion liquid can be obtained by pulverizing treatment of the cellulose or the cellulose derivative, preferably obtained by physical pulverization. The physical pulverization is carried out by applying a physical external force to a mixture of the cellulose or the cellulose derivative and a dispersing medium using a stirring apparatus such as a magnetic stirrer, a stirring blade, etc., a homogenizer such as a polytron, etc., an ultrasonic generating apparatus such as an ultrasonic crusher, etc., a pulverizer such as a wet-type pulverization apparatus (for example, Star Brust; SUGINO MACHINE LIMITED), etc. However, if the commercially available cellulose or the cellulose derivative is a material sufficiently pulverized, the dispersion liquid may be obtained without carrying out the pulverization treatment. Also, in the producing method of the present invention, in place of the step of obtaining the dispersion liquid, a commercially available cellulose dispersion liquid, for example, a commercially available cellulose nanofiber dispersion liquid may be used.

The particles of the present invention are obtained by subjecting the obtained dispersion liquid to spray-drying. Spray-drying is carried out using a known spray-drying device such as a spray dryer, etc. Spray-drying conditions are appropriately set depending on the kind of the dispersing medium in the dispersion liquid, the kind or the concentration of the cellulose or the cellulose derivative, etc., and, for example, are carried out at an inlet temperature of 150 to 300°C and an outlet temperature of 0 to 150°C.

<Cosmetics>

In the particle of the present invention, the crystallization degree is appropriately suppressed, and it has a wrinkle-like or fold-like uneven structure on the surface thereof so that it is soft, and has excellent optical characteristics (light scattering properties) that the incident light is uniformly light scattered, so that it is suitable for adding to cosmetics that directly touch the skin and require optical properties such as a defocus effect. Also, the particle of the present invention may be utilized as an additive for cosmetics or a composition for cosmetics by itself or in the form of being dispersed together with any additive in a suitable medium depending on the application. Examples of such cosmetics may be mentioned toiletry products such as face wash foams, face wash powders, body wash products, etc., hair care products such as shampoos, conditioners, etc., oral care products such as dentifrices, etc., makeup cosmetics such as makeup bases, powder foundations, liquid foundations, BB creams, concealer, sunscreen, etc., and these can be used as a scrubbing agent for enhancing a massage effect or a cleaning effect, or as a light scattering agent for expressing a defocusing effect, etc. In particular, since the particles of the present invention are excellent in oil absorption, they are suitable for adding to cosmetics containing an oily component(s) and an oily base(s).

<Other additives>

To the cosmetics of the present invention, an additive(s) generally usable as an additive(s) for cosmetics and an additive for quasi drugs may be formulated depending on necessity. As an additive component(s) such as a physiologically active substance(s) and a functional substance(s), etc., formulated to an external preparation(s) for skin such as cosmetics or quasi drugs, etc., there may be mentioned, for example, oily bases, moisturizers, touch improvers, surfactants, polymers, thickening or gelling agents, solvents, propellants, antioxidants, reducing agents, oxidizing agents, antiseptic agents, antibacterial agents, bactericidal agents, chelating agents, pH adjusting agents, acids, alkalis, powders, inorganic salts, ultraviolet absorber, whitening agents, vitamins and its derivatives, hair-growth agents, blood circulation promoters, stimulants, hormones, anti-wrinkle agents, anti-aging agents, tightening agents, cooling sensation agents, thermal sensation agents, wound healing accelerators, irritation mitigating agents, analgesics, cell activators, plant/animal/microbial extracts, antipruritic agents, keratin exfoliating/dissolving agents, antiperspirants, algefacients, astringents, enzymes, nucleic acids, fragrances, pigments, coloring agents, dyes, pigments, antiphlogistics, anti-inflammatory agents, anti-asthmatic agents, anti-chronic obstructive pulmonary disease agents, anti-allergic agents, immunomodulators, anti-infective agents and antifungal agents, etc.

When these additive components are exemplified, there may be mentioned as preferable examples including, as an oily base, higher (polyvalent) alcohols such as cetanol, myristyl alcohol, oleyl alcohol, lauryl alcohol, cetostearyl alcohol, stearyl alcohol, arachidyl alcohol, behenyl alcohol, jojoba alcohol, chimyl alcohol, selachyl alcohol, batyl alcohol, hexyldecanol, isostearyl alcohol, 2-octyldodecanol, dimer diol, etc.; aralkyl alcohol such as benzyl alcohol, etc., and its derivatives; higher fatty acids such as lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisohenicosanoic acid, long-chain branched fatty acid, dimer acid, hydrogenated dimer acid, etc., and metal soaps thereof such as aluminum salt, calcium salt, magnesium salt, zinc salt, potassium salt, sodium salt, etc., and nitrogen-containing derivatives thereof such as amides; hydrocarbons such as liquid paraffin (mineral oil), heavy liquid isoparaffin, light liquid isoparaffin, $\alpha$-olefin oligomer, polyisobutene, hydrogenated polyisobutene, polybutene, squalane, olive-derived squalane, squalene, vaseline, solid paraffin, etc.; waxes such as candelilla wax, carnauba wax, rice wax, wood wax, beeswax, montan wax, ozokerite, ceresin, paraffin wax, microcrystalline wax, petrolatum, Fischer-Tropsch wax, polyethylene wax, ethylenepropylene copolymer, etc.; vegetable oils such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadow foam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, hydrogenated jojoba oil, etc.; animal oils such as beef tallow, milk fat, horse fat, egg yolk oil, mink oil, turtle oil, etc.; animal waxes such as whale wax, lanolin, orange roughy oil, etc.; lanolins such as liquid lanolin, reduced lanolin, adsorption refined lanolin, lanolin acetate, acetic acid liquid lanolin, hydroxy lanolin, polyoxyethylene lanolin, lanolin fatty acid, hard lanolin fatty acid, lanolin alcohol, acetylated lanolin alcohol, acetic acid (cetyl-lanolyl) ester, etc.; sphingophospholipids such as lecithin, phosphatidylcholine, phosphatidylethanolamine, phosphatidylserine, phosphatidylglycerol, phosphatidylinositol, sphingomyelin, etc., phospholipids such as phosphatidic acid, lysolecithin, etc.; phospholipid derivatives such as hydrogenated soybean phospholipids, partially hydrogenated soybean phospholipids, hydrogenated egg yolk phospholipids, partially hydrogenated egg yolk phospholipids, etc.; sterols such as cholesterol, dihydrocholesterol, lanosterol, dihydrolanosterol, phytosterol, cholic acid, etc.; sapogenins; saponins; acylsarcosine alkyl esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, isopropyl N-lauroylsarcosine, etc., sterol esters such as cholesteryl 12-hydroxystearate, cholesteryl macadamia nut oil fatty acid, phytosteryl macadamia nut oil fatty acid, phytosteryl isostearate, cholesteryl soft lanolin fatty acid, cholesteryl hard lanolin fatty acid, cholesteryl long-chain branched fatty acid, cholesteryl long-chain $\alpha$-hydroxy fatty acid, etc.; lipid complexes such as phospholipid-cholesterol complex, phospholipid-phytosterol complex, etc.; monoalcohol carboxylic acid esters such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolin fatty acid, hexyldecyl dimethyloctanoate, octyldodecyl ercucate, hydrogenated castor oil isostearate, ethyl oleate, avocado oil fatty acid ethyl, isopropyl myristate, isopropyl palmitate, octyl palmitate, isopropyl isostearate, lanolin fatty acid isopropyl, diethyl sebacate, diisopropyl sebacate, dioctyl sebacate, diisopropyl adipate, dibutyloctyl sebacate, diisobutyl adipate, dioctyl succinate, triethyl citrate, etc.; oxyacid esters such as cetyl lactate, diisostearyl malate, hydrogenated castor oil monoisostearate, etc.; polyhydric alcohol fatty acid esters such as glyceryl trioctanoate, glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosadiate, trimethylolpropane trioctanoate, trimethylolpropane triisostearate, neopentyl glycol dioctanoate, neopentyl glycol dicaprate, 2-butyl-2-ethyl-1,3-propane diol dioctanoate, propylene glycol dioleate, pentaerythrityl tetraoctanoate, hydrogenated rosin pentaerythrityl, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, tetraisostearate polyglyceryl, polyglyceryl-10 nonaisostearate, polyglyceryl-8 deca(ercucate/isostearate/ricinoleate), (hexyldecanoic acid/sebacic acid) diglyceryl oligo ester, glycol distearate (ethylene glycol distearate), 3-methyl-1,5-pentanediol dineopentanoate, 2,4-diethyl-1,5-pentanediol dineopentanoate, etc.; derivatives of dimer acid or dimer diol such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, dimer dilinoleic acid hydrogenated castor oil dimer dilinoleate, hydroxyalkyl dimer dilinoleyl ether, etc.; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (paltamide MEA), palmitic acid diethanolamide (paltamide DEA), coconut oil fatty acid methylethanolamide (cocamide methyl MEA), etc.; dimethicone(dimethylpolysiloxane), highly polymerized dimethicone (highly polymerized dimethylpolysiloxane), cyclomethicone (cyclic dimethylsiloxane, decamethylcyclopentasiloxane), phenyltrimethicone, diphenyldimethicone, phenyldimethicone, (aminoethyl aminopropyl-methicone/dimethicone)copolymer, dimethiconol, dimethiconol crosspolymer, silicone resin, silicone rubber, amino-modified silicones such as aminopropyldimethicone and amodimethicone, etc., cation-modified silicone, polyether-modified silicones such as dimethicone copolyol, etc., silicones such as polyglycerin-modified silicone, sugar-modified silicone, carboxylic acid-modified silicone, phosphoric acid-modified silicone, sulfuric acid-modified silicone, alkyl-modified silicone, fatty acid-modified silicone, alkyl ether-modified silicone, amino acid-modified silicone, peptide-modified silicone, fluorine-modified silicone, cation-modified and polyether-modified silicone, amino-modified and polyether-modified silicone, alkyl-modified and polyether-modified silicone, polysiloxane-oxyalkylene copolymer, etc.; fluorine-based oil agents such as perfluorodecane, perfluorooctane, perfluoropolyether, etc.

As the moisturizers and touch improvers, there may be mentioned as preferable examples including polyols and its polymerized products such as glycerin, 1,3-butylene glycol, propylene glycol, 3-methyl-1,3-butane diol, 1,3-propane diol, 2-methyl-1,3-propane diol, trimethylolpropane, pentaerythritol, hexylene glycol, diglycerin, polyglycerin, diethylene glycol, polyethylene glycol, dipropylene glycol, polypropylene glycol, ethylene glycol-propylene glycol copolymer, etc.; glycol alkyl ethers such as diethylene glycol monoethyl ether (ethoxydiglycol), ethylene glycol monoethyl ether, ethylene glycol monobutyl ether, diethylene glycol dibutyl ether, etc.; water-soluble esters such as polyglyceryl-10 (eicosanedioate/tetradecanedioate), polyglyceryl-10 tetradecanedioate, etc.; sugar alcohols such as sorbitol, xylitol, erythritol, mannitol, maltitol, etc.; saccharides and its derivatives such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, γ-cyclodextrin, and modified cyclodextrins such as maltosylated, hydroxyalkylated, etc.), β-glucan, chitin, chitosan, heparin and derivatives, pectin, arabinogalactan, dextrin, dextran, glycogen, ethyl glucoside, glucosylethyl methacrylate polymerized products or copolymerized products, etc.; hyaluronic acid, sodium hyaluronate; sodium chondroitin sulfate; mucoitinsulfuric acid, caronine sulfate, keratosulfate, dermatan sulfate; Tremella fuciformis (snow fungus) extract, Tremella fuciformis (snow fungus) polysaccharides; fucoidan; polianthes tuberosa polysaccharide or naturally occurring polysaccharides; organic acids such as citric acid, tartaric acid, lactic acid, etc., and its salts; urea and its derivatives; 2-pyrrolidone-5-carboxylic acid and its salt such as sodium, etc.; amino acids and salts thereof such as betaine (trimethylglycine), proline, hydroxy proline, arginine, lysine, serine, glycine, alanine, phenylalanine, tyrosine, β-alanine, threonine, glutamic acid, glutamine, asparagine, aspartic acid, cystine, cysteine, methionine, leucine, isoleucine, valine, tryptophan, histidine, taurine, etc.; protein peptides and its derivatives such as collagen, fish-derived collagen, atelocollagen, gelatin, elastin, collagen-degraded peptide, hydrolyzed collagen, hydroxypropylammonium chloride hydrolyzed collagen, elastin-degraded peptide, keratin-degraded peptide, hydrolyzed keratin, conchiolin-degraded peptide, hydrolyzed conchiolin, silk protein-degraded peptide, hydrolyzed silk, lauroyl hydrolyzed silk sodium, soybean protein-degraded peptide, wheat protein-degraded peptide, hydrolyzed wheat protein, casein-degraded peptide, acylated peptide, etc.; acylated peptides such as palmitoyl oligopeptide, palmitoyl penta peptide, palmitoyl tetrapeptide, etc.; silylated peptides; animal and plant extract components such as lactic acid bacteria culture fluid, yeast extract, egg shell membrane protein, bovine submaxillary mucin, hypotaurine, sesame lignan glycosides, glutathione, albumin, milk serum; choline chloride, phosphorylcholine; placenta extract, aerastin, collagen, aloe extract, hamamelis water, loofah water, chamomile extract, licorice extract, comfrey extract, silk extract, Rosa roxburghii fruit extract, Achillea Millefolium extract, eucalyptus extract, melilot extract, etc., ceramides such as natural ceramides (types 1, 2, 3, 4, 5 and 6), hydroxyceramides, pseudoceramides, glycosphingolipids, ceramides and ceramide saccharide-containing extract, etc.

As the surfactants, nonionic surfactants, nonionic surfactants, cationic surfactants, amphoteric surfactants, polymers surfactants, etc., are mentioned as preferable examples. When preferred materials as the surfactants are exemplified, as the nonionic surfactants, there may be mentioned as preferable examples including fatty acid salts such as sodium laurate, sodium myristate, sodium palmitate, sodium stearate, potassium laurate, potassium myristate, etc.; alkyl sulfuric acid ester salts such as sodium lauryl sulfate, lauryl sulfate triethanolamine, ammonium lauryl sulfate, etc.; polyoxyethylene alkylsulfates such as sodium laureth sulfate, triethanolamine laureth sulfate, etc.; acyl N-methylamino acid salts such as sodium methyl cocoyl taurate, potassium methyl cocoyl taurate, sodium methyl lauroyl taurate, sodium methyl myristoyl taurate, sodium methyl lauroyl alanine, sodium lauroyl sarcosine, triethanolamine lauroylsarcosinate, sodium alanine methyl lauroyl glutamate, etc.; acyl amino acid salts such as sodium cocoyl glutamate, triethanolamine cocoyl glutamate, sodium lauroyl glutamate, sodium myristoyl glutamate, sodium stearoyl glutamate, ditriethanolamine palmitoyl asparatate, triethanolamine cocoylalaninate, etc.; polyoxyethylene alkyl ether acetates such as sodium laureth acetate, etc.; succinic acid ester salts such as sodium lauroyl monoethanolamide succinate, etc.; fatty acid alkanolamide ether carboxylic acid salts; acyl lactates; polyoxyethylene aliphatic amine sulfates; fatty acid alkanolamide sulfates; fatty acid glyceride sulfates such as sodium hardened coconut oil fatty acid glycerin sulfate, etc.; alkylbenzene polyoxyethylene sulfates; olefin sulfonates such as sodium α-olefin sulfonate, etc.; alkyl sulfosuccinates such as lauryl 2 sodium sulfosuccinate, dioctyl sodium sulfosuccinate, etc.; alkyl ether sulfosuccinates such as laureth 2 sodium sulfosuccinate, monolauroyl monoethanolamide sodium polyoxyethylene sulfosuccinate, sodium lauryl polypropylene glycol sulfosuccinate, etc.; alkylbenzene sulfonates such as sodium tetradecylbenzene sulfonate, triethanolamine tetradecylbenzene sulfonate, etc.; alkylnaphthalene sulfonates; alkane sulfonates; α-sulfo fatty acid methyl ester salt; acyl isethionates; alkyl glycidyl ether sulfonates; alkyl sulfoacetates; alkyl ether phosphoric acid ester salts such as sodium laureth phosphate, sodium dilaureth phosphate, sodium trilaureth phosphate, sodium monooleth phosphate, etc.; alkyl phosphoric acid ester salts such as potassium lauryl phosphate, etc.; sodium caseinate; alkyl aryl ether phosphates; fatty acid amide ether phosphates; phospholipids such as phosphatidyl glycerol, phosphatidyl inositol, phosphatidic acid, etc.; silicone-based anionic surfactants such as carboxylic acid-modified silicone, phosphoric acid-modified silicone, sulfuric acid-modified silicone, etc.; the nonionic surfactants including polyoxyethylene alkyl ethers with various polyoxyethylene addition numbers such as laureth (polyoxyethylene lauryl ether), ceteth (polyoxyethylene cetyl ether), steareth (polyoxyethylene stearyl ether), behenes (polyoxyethylene behenyl ether), isosteareth (polyoxyethylene isostearyl ether), octyldodecethes (polyoxyethylene octyldodecyl ether), etc.; polyoxyethylene alkylphenyl ethers; castor oil and hydrogenated castor oil derivatives such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamic acid monoisostearate diester, polyoxyethylene hydrogenated castor oil maleic acid, etc.; polyoxyethylene phytosterols; polyoxyethylene cholesterols; polyoxyethylene cholestanols; polyoxyethylene lanolin; polyoxyethylene reduced lanolin; polyoxyethylene-polyoxypropylene alkyl ethers such as polyoxyethylene-polyoxypropylene cetyl ether, polyoxyethylene-polyoxypropylene 2-decyl tetradecyl ether, polyoxyethylene-polyoxypropylene monobutyl ether, polyoxyethylene-polyoxypropylene hydrogenated lanolin, polyoxyethylene-polyoxypropylene glycerin ether, etc.; polyoxyethylene-polyoxypropylene glycols; (poly)glycerin polyoxypropylene glycols such as PPG-9 diglyceryl, etc.; glycerin fatty acid partial esters such as glyceryl stearate, glyceryl isostearate, glyceryl palmitate, glyceryl myristate, glyceryl oleate, glyceryl coconut oil fatty acid, glycerin monocotton seed oil fatty acid, glycerin monoercucate, glycerin sesquioleate, glycerin $\alpha,\alpha$'-oleic acid pyroglutamate, glycerin malic acid monostearate, etc.; polyglycerin fatty acid esters such as polyglyceryl-2 stearate, ditto 3, ditto 4, ditto 5, ditto 6, ditto 8, ditto 10, polyglyceryl-6 distearate, ditto 10, polyglyceryl-2 tristearate, polyglyceryl-10 decastearate, polyglyceryl-2 isostearate, ditto 3, ditto 4, ditto 5, ditto 6, ditto 8, ditto 10, polyglyceryl-2 (diglyceryl diisostearate) diisostearate, ditto 3, ditto 10, polyglyceryl-2 triisostearate, polyglyceryl-2 tetraisostearate, polyglyceryl-10 decaisostearate, polyglyceryl-2 oleate, ditto 3, ditto 4, ditto 5, ditto 6, ditto 8, ditto 10, polyglyceryl-6 dioleate, polyglyceryl-2 trioleate, polyglyceryl-10 decaoleate, etc.; ethylene glycol monofatty acid esters such as ethylene glycol monostearate, etc.; propylene glycol monofatty acid esters such as propylene glycol monostearate, etc.; pentaerythritol partial fatty acid esters; sorbitol partial fatty acid esters; maltitol partial fatty acid esters; maltitol ether; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, diglycerol sorbitan tetra-2-ethylhexylate, etc.; sugar derivative partial esters such as sucrose fatty acid ester, methyl glucoside fatty acid ester, trehalose undecylenate, etc.; alkyl glucosides such as caprylyl glucoside, etc.; alkyl polyglycosides; lanolin alcohol; reduced lanolin; polyoxyethylene fatty acid mono- and diesters such as polyoxyethylene distearate, polyethylene glycol diisostearate, polyoxyethylene monooleate, polyoxyethylene dioleate, etc.; polyoxyethylene-propylene glycol fatty acid esters; polyoxyethylene glycerin fatty acid esters such as polyoxyethylene glycerin monostearate, polyoxyethylene glycerin monoisostearate, polyoxyethylene glycerin triisostearate, etc., polyoxyethylene monooleate, etc.; polyoxyethylene sorbitan fatty acid esters such as polyoxyethylene sorbitan monooleate, polyoxyethylene sorbitan monostearate, polyoxyethylene sorbitan monooleate, polyoxyethylene sorbitan tetraoleate, etc.; polyoxyethylene sorbitol fatty acid esters such as polyoxyethylene sorbitol monolaurate, polyoxyethylene sorbitol monooleate, polyoxyethylene sorbitol pentaoleate, polyoxyethylene sorbitol monostearate, etc.; polyoxyethylene methylglucoside fatty acid esters; polyoxyethylene alkyl ether fatty acid esters; polyoxyethylene animal and vegetable oils and fats such as polyoxyethylene sorbitol bees wax, etc.; alkyl glyceryl ethers such as isostearyl glyceryl ether, chimyl alcohol, selachyl alcohol, batyl alcohol, etc.; polyhydric alcohol alkyl ethers; polyoxyethylene alkylamines; tetrapolyoxyethylene-tetrapolyoxypropylene-ethylenediamine condensates; natural surfactants such as saponin, sophorolipid, etc.; polyoxyethylene fatty acid amides; fatty acid alkanolamides such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (paltamide MEA), palmitic acid diethanolamide (paltamide DEA), coconut oil fatty acid methylethanolamide (cocamide methyl MEA), etc.; alkyldimethylamine oxides such as lauramin oxide, cocamine oxide, stearamine oxide, behenamine oxide, etc.; alkylethoxydimethylamine oxides; polyoxyethylene alkyl mercaptans; polyether-modified silicones such as dimethicone copolyol, etc., silicone-based nonionic surfactants such as polysiloxane-oxyalkylene copolymer, polyglycerin-modified silicone, sugar-modified silicone, etc.; as the cationic surfactants, there may be mentioned alkyl trimethylammonium chlorides such as behentrimonium chloride, steartrimonium chloride, cetrimonium chloride, lauryl trimonium chloride, etc.; alkyl trimethylammonium bromides such as stearyltrimonium bromide, etc.; dialkyldimethylammonium chlorides such as distearyldimonium chloride, dicocodimonium chloride, etc.; fatty acid amide amines and a salt thereof such as stearamidopropyl dimethylamine, stearamidoethyl diethylamine, etc.; alkyl ether amines such as stearoxypropyl dimethylamine, and salts thereof or quaternary salts, etc.; fatty acid amide type quaternary ammonium salts such as ethyl sulfate long-chain branched fatty acid (12 to 31) aminopropylethyl dimethylammonium, ethyl sulfate lanolin fatty acid aminopropylethyl dimethylammonium, etc.; polyoxyethylenealkylamines and salts thereof or quaternary salts; alkylamine salts; fatty acid amide guanidinium salts; alkyl ether ammonium salts; alkyl trialkylene glycol ammonium salts; benzalkonium salts; benzethonium salts; pyridinium salts such as cetylpyridinium chloride; imidazolinium salts; alkylisoquinolinium salts; dialkylmorpholinium salts; polyamine fatty acid derivatives; amino-modified silicones such as aminopropyl dimethicone and amodimethicone, etc., silicone-based cationic surfactants such as cation-modified silicones, cation-modified and polyether-modified silicones, amino-modified and polyether-modified silicones, etc.; as the amphoteric surfactants, there may be mentioned N-alkyl-N,N-dimethylamino acid betaines such as lauryl betaine (lauryl dimethylaminoacetic betaine), etc.; fatty acid amide alkyl-N,N-dimethylamino acid betaines such as cocamidopropyl betaine, lauramidopropyl betaine, etc.; imidazoline type betaines such as sodium cocoamphoacetate, sodium lauroamphoacetate, etc.; alkylsulfobetaines such as alkyldimethyltaurine, etc.; sulfate type betaines such alkyldimethylaminoethanol sulfate, etc.; phosphate type betaines such as alkyldimethylaminoethanol phosphate, etc.; sphingophospholipids such as phosphatidylcholine, phosphatidylethanolamine, phosphatidylserine, sphingomyelin, etc., lysolecithin, phospholipids such as hydrogenated soybean phospholipid, partially hydrogenated soybean phospholipid, hydrogenated egg yolk phospholipid, partially hydrogenated egg yolk phospholipid, hydroxylated lecithin, etc.; silicone-based amphoteric surfactants, etc.; as the polymer surfactants, there may be mentioned polyvinyl alcohol, sodium alginate, starch derivatives, tragacanth gum, acrylic acid-alkyl methacrylate copolymer; silicone-based various kinds of surfactants as preferable materials.

As the polymers, thickening agents and gelling agents, there may be mentioned guar gum, locust bean gum, quince seed, carrageenan, galactan, gum arabic, tara gum, tamarind, furcellaran, karaya gum, sunset hibiscus, cara gum, tragacanth gum, pectin, pectic acid and a salt such as sodium salt, etc., alginic acid and a salt such as sodium salt, etc., mannan; starches such as rice, corn, potato, wheat, etc.; xanthan gum, dextran, succinoglucan, curdlan, hyaluronic acid and a salt thereof, xanthan gum, pullulan, gellan gum, chitin, chitosan, agar, brown algae extract, chondroitin sulfate, casein, collagen, gelatin, albumin; celluloses and their derivatives such as methyl cellulose, ethyl cellulose, hydroxyethyl cellulose, hydroxypropyl cellulose, hydroxypropylmethyl cellulose, carboxymethyl cellulose and a salt such as sodium, etc., methyl hydroxypropyl cellulose, sodium cellulose sulfate, dialkyl dimethyl ammonium sulfate cellulose, crystalline cellulose, cellulose powder, etc.; starch-based polymers such as soluble starch, carboxymethyl starch, methyl hydroxypropyl starch, methyl starch, etc., starch derivatives such as starch hydroxypropyltrimonium chloride, corn starch aluminum octenyl succinate, etc.; alginic acid derivatives such as sodium alginate, alginate propylene glycol ester, etc.; polyvinylpyrrolidone (PVP), polyvinyl alcohol (PVA), vinylpyrrolidone-vinyl alcohol copolymer, polyvinylmethylether; polyethylene glycol, polypropylene glycol, polyoxyethylene-polyoxypropylene copolymer; amphoteric methacrylic acid ester copolymers such as (methacryloyloxyethyl carboxy betaine/alkyl methacrylate) copolymers, (acrylates/stearyl acrylate/ethyl methacrylate amine oxide) copolymers, etc.; (dimethicone/vinyl dimethicone) crosspolymers, (alkyl acrylate/diacetone acrylamide) copolymers, (alkyl acrylate/diacetone acrylamide) copolymer AMP; polyvinyl acetate partially saponified products, maleic acid copolymers; vinyl pyrrolidone-dialkylaminoalkyl methacrylate copolymers; acrylic resin alkanolamine; polyester, water-dispersible polyester; polyacrylamide; polyacrylic acid ester copolymers such as polyethyl acrylate, etc., carboxyvinyl polymers, polyacrylic acid and salts such as sodium salt, etc., acrylic acid-methacrylic acid ester copolymers; acrylic acid-alkyl methacrylate copolymers; cationized cellulose such as polyquaternium-10, etc., diallyl dimethyl ammonium chloride-acrylamide copolymers such as polyquaternium-7, etc., acrylic acid-diallyl dimethyl ammonium chloride copolymers such as polyquaternium-22, etc., acrylic acid-diallyl dimethyl ammonium chloride-acrylamide copolymers such as polyquaternium-39, etc., acrylic acid-cationized methacrylic acid ester copolymers, acrylic acid-cationized methacrylamide copolymers, acrylic acid-methyl acrylate-methacrylamide propyltrimethyl ammonium chloride copolymers such as polyquaternium-47, etc., methacryloyl chloride choline ester polymers; cationized oligosaccharides, cationized dextran, cationized polysaccharides such as guar hydroxypropyltrimonium chloride, etc.; polyethylene-imine; cation polymer; 2-methacryloyloxyethyl phosphorylcholine polymers such as polyquaternium-51, etc. and copolymers with butyl methacrylate copolymer, etc.; polymeric emulsions such as acrylic resin emulsion, polyethyl acrylate emulsion, polyacrylic alkyl ester emulsion, polyvinyl acetate resin emulsion, natural rubber latex, synthetic latex, etc.; nitrocellulose; polyurethanes and various kinds of copolymers; various kinds of silicones; silicone-based various kinds of copolymers such as acryl-silicone grafted copolymers, etc.; various kinds of fluorine-based polymers; 12-hydroxystearic acid and a salt thereof; dextrin fatty acid esters such as dextrin palmitate, dextrin myristate, etc.; silicic anhydride, fumed silica (ultrafine particle silicic anhydride), magnesium aluminum silicate, sodium magnesium silicate, metallic soap, dialkylphosphate metal salt, bentonite, hectorite, organic-modified clay minerals, sucrose fatty acid esters, and fructooligosaccharide fatty acid esters as preferable materials. Among the above examples, cellulose and its derivatives, alginic acid and its salts, polyvinyl alcohol, hyaluronic acid and its salts, or collagen are preferable.

As the solvents, there may be mentioned lower alcohols such as ethanol, 2-propanol (isopropyl alcohol), butanol, isobutyl alcohol, etc.; glycols such as propylene glycol, 1,3-butylene glycol, diethylene glycol, dipropylene glycol, isopentyl diol, etc.; glycol ethers such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, dipropylene glycol monoethyl ether, etc.; glycol ether esters such as ethylene glycol monoethyl ether acetate, diethylene glycol monoethyl ether acetate, propylene glycol monoethyl ether acetate, etc.; glycol esters such as diethoxyethyl succinate, ethylene glycol disuccinate, etc.; benzyl alcohol, benzyloxyethanol, propylene carbonate, dialkyl carbonate, acetone, ethyl acetate, N-methylpyrrolidone; toluene, etc., and as the propellants, there may be mentioned fluorocarbon, next-generation Freon; LPG, dimethyl ether, carbon dioxide, etc., as preferable materials.

As the antioxidants, there may be mentioned tocopherol derivatives such as tocopherol (vitamin E), tocopherol acetate, etc.; BHT, BHA; gallic acid derivatives such as propyl gallate, etc.; vitamin C (ascorbic acid) and/or its derivatives; erythorbic acid and its derivatives; sulfurous acid salts such as sodium sulfite, etc.; hydrogen sulfite such as sodium hydrogen sulfite, etc.; thiosulfate such as sodium thiosulfate, etc.; metabisulfite; thiotaurine, hypotaurine; thioglycerol, thiourea, thioglycolic acid, and cysteine hydrochloride as preferable materials.

As the reducing agents, there may be mentioned thioglycolic acid, cysteine, cysteamine, etc., as preferable materials.

As the oxidizing agents, there may be mentioned hydrogen peroxide solution, ammonium persulfate, sodium bromate, percarbonate, etc., as preferable materials.

As the antiseptic agents, antibacterial agents and bactericidal agents, there may be mentioned hydroxybenzoic acid and salts thereof or esters thereof such as methylparaben, ethylparaben, propylparaben, butylparaben, etc.; salicylic acid; sodium benzoate; phenoxyethanol; isothiazolinone derivatives such as methylchloroisothiazolinone, methylisothiazolinone, etc.; imidazolinium urea; dehydroacetic acid and its salts; phenols; halogenated bisphenols such as triclosan, etc., acid amides, quaternary ammonium salts; trichlorocarbanide, zinc pyrithione, benzalkonium chloride, benzethonium chloride, sorbic acid, chlorhexidine, chlorhexidine gluconate, halocarban, hexachlorophene, hinokitiol; the other phenols such as phenol, isopropylphenol, cresol, thymol, parachlorophenol, phenylphenol, sodium phenylphenol, etc.; phenylethyl alcohol, photosensitizers, antibacterial zeolite, and silver ion, as preferable materials.

As the chelating agents, there may be mentioned edetates (ethylenediamine tetraacetate) such as EDTA, EDTA2Na, EDTA3Na, EDTA4Na, etc.; hydroxyethyl ethylenediamine triacetate such as HEDTA3Na, etc.; pentetic acid salt (diethylenetriamine pentaacetate); phytic acid; phosphonic acid such as etidronic acid, etc., and salts thereof such as sodium salt, etc.; sodium oxalate; polyamino acids such as polyaspartic acid, polyglutamic acid, etc.; poly sodium phosphate, sodium metaphosphate, phosphoric acid; sodium citrate, citric acid, alanine, dihydroxyethyl glycine, gluconic acid, ascorbic acid, succinic acid, and tartaric acid as preferable materials.

As the pH adjusting agents, acids and alkalis, there may be mentioned citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propane diol, 2-amino-2-hydroxymethyl-1,3-propane diol, arginine, sodium hydroxide, potassium hydroxide, aqueous ammonia, guanidine carbonate, and ammonium carbonate as preferable materials.

As the powders, there may be mentioned inorganic powder including inorganic powder with various kinds of sizes and shapes such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate metal salt, magnesium, zeolite, barium sulfate, calcined calcium sulfate, calcium phosphate, fluoroapatite, hydroxyapatite, ceramic powder, bentonite, smectite, clay, mud, metallic soap (for example, zinc myristate, calcium palmitate, aluminum stearate), calcium carbonate, red iron oxide, yellow iron oxide, black iron oxide, ultramarine, Prussian blue, carbon black, titanium oxide, fine particles and ultrafine particle titanium oxide, zinc oxide, fine particles and ultrafine particles zinc oxide, alumina, silica, fumed silica(ultrafine particle silicic anhydride), titanated mica, fish scale, boron nitride, photochromic pigments, synthetic fluorine phlogopite, fine particle complex powder, gold, aluminum, etc., and powder which is made hydrophobic or hydrophilic by subjecting these powder to the treatment with various kinds of surface treatment agents including silicone such as hydrogen silicone, cyclic hydrogen silicone, etc., or the other silane or titanium coupling agents, etc.; organic-based powder with various kinds of sizes and shapes and surface treated powder such as starch, cellulose, Nylon powder, polyethylene powder, polymethyl methacrylate powder, polystyrene powder, copolymer resin powder of styrene and acrylic acid, polyester powder, benzoguanamine resin powder, polyethylene terephthalate-polymethyl methacrylate laminated powder, polyethylene terephthalate-aluminum-epoxy laminated powder, etc., urethane powder, silicone powder, Teflon (Registered Trademark) powder, etc., and organic and inorganic complex powder as preferable materials.

As the inorganic salts, there may be mentioned sodium chloride-containing salts such as common salt, normal salt, rock salt, sea salt, natural salt, etc.; potassium chloride, aluminum chloride, calcium chloride, magnesium chloride, bittern, zinc chloride, ammonium chloride; sodium sulfate, aluminum sulfate, aluminum sulfate-potassium (alum), aluminum sulfate-ammonium, barium sulfate, calcium sulfate, potassium sulfate, magnesium sulfate, zinc sulfate, iron sulfate, copper sulfate; sodium phosphates such as 1Na-2Na-3Na phosphate, etc., potassium phosphates, calcium phosphates, and magnesium phosphates as preferable materials.

As the ultraviolet absorbers, there may be mentioned benzoic acid-based ultraviolet absorbers such as para-aminobenzoic acid, para-aminobenzoic acid monoglycerin ester, N,N-dipropoxy-para-aminobenzoic acid ethyl ester, N,N-diethoxypara-aminobenzoic acid ethyl ester, N,N-dimethyl-para-aminobenzoic acid ethyl ester, N,N-dimethyl-para-aminobenzoic acid butyl ester, N,N-dimethyl-para-aminobenzoic acid ethyl ester, etc.; anthranilic acid-based ultraviolet absorbers such as homomenthyl-N-acetylanthranilate, etc.; salicylic acid-based ultraviolet absorbers such as salicylic acid and its sodium salt amyl salicylate, menthyl salicylate, homomenthyl salicylate, octyl salicylate, phenyl salicylate, benzyl salicylate, p-isopropanolphenyl salicylate, etc.; cinnamic acid-based ultraviolet absorbers such as octyl cinnamate, ethyl-4-isopropylcinnamate, methyl-2,5-diisopropylcinnamate, ethyl-2,4-diisopropyl-cinnamate, methyl-2,4-diisopropyl-cinnamate, propyl-p-methoxycinnamate, isopropyl-p-methoxycinnamate, isoamyl-p-methoxycinnamate, 2-ethylhexyl p-methoxycinnamate (octyl paramethoxycinnamate), 2-ethoxyethyl-p-methoxycinnamate (cinoxate), cyclohexyl-p-methoxycinnamate, ethyl-$\alpha$-cyano-$\beta$-phenylcinnamate, 2-ethylhexyl $\alpha$-cyano-$\beta$-phenylcinnamate (octocrylene), glyceryl mono-2-ethylhexanoyl-dipara-methoxycinnamate, ferulic acid and its derivatives, etc.; benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.; 3-(4'-methylbenzylidene)-d,l-camphor, 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 5-(3,3-dimethyl-2-norbornylidene)-3-pentan-2-one; dibenzoylmethane derivatives such as 4-t-butylmethoxydibenzoylmethane, etc.; octyl triazone; urocanic acid derivatives such as urocanic acid and ethyl urocanate, etc.; hydantoin derivatives such as 2-(2'-hydroxy-5'-methylphenyl)benzotriazole, 1-(3,4-dimethoxyphenyl)-4,4-dimethyl-1,3-pentanedione, 2-ethylhexyl dimethoxybenzylidenedioxoimidazolidine propionate, etc., phenylbenzimidazole sulfonic acid, terephthalylidene dicamphor sulfonic acid, drometrizole trisiloxane, methyl anthranilate, rutin and its derivatives, and oryzanol and its derivatives as preferable materials.

As the whitening agents, there may be mentioned hydroquinone glycosides and their esters such as arbutin, α-arbutin, etc.; ascorbic acid phosphoric acid ester salts such as ascorbic acid, ascorbic acid phosphoric acid ester sodium salt and ascorbic acid phosphoric acid ester magnesium salt, etc., ascorbic acid fatty acid esters such as ascorbic acid tetraisopalmitic acid ester, etc., ascorbic acid alkyl ethers such as ascorbic acid ethyl ether, etc., ascorbic acid glucoside and fatty acid esters thereof such as ascorbic acid-2-glucoside, etc., ascorbic acid derivatives such as ascorbic acid sulfuric acid ester, tocopheryl ascorbyl phosphate, etc.; kojic acid, ellagic acid, tranexamic acid and its derivatives, ferulic acid and its derivatives, and plant extracts such as placenta extract, glutathione, oryzanol, butylresorcinol, oil-soluble chamomile extract, oil-soluble licorice extract, Chinese tamarisk extract, saxifraga extract, etc., as preferable materials.

As the vitamins and its derivatives, there may be mentioned vitamin A such as retinol, retinol acetate, retinol palmitate, etc.; thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acids, nicotinic acids such as nicotinamide-benzyl nicotinate, etc., vitamin B group such as cholines, etc.; vitamin C such as ascorbic acid and a salt of sodium, etc.; vitamin D; vitamin E such as α, β, γ and δ-tocopherol, etc.; the other vitamins such as pantothenic acid, biotin, etc.; ascorbic acid phosphoric acid ester salts such as sodium salt of ascorbic acid phosphoric acid ester and magnesium salt of ascorbic acid phosphoric acid ester, etc., ascorbic acid fatty acid esters such as ascorbic acid tetraisopalmitic acid ester, ascorbyl stearate, ascorbyl palmitate, ascorbyl dipalmitate, etc., ascorbic acid alkyl ethers such as ascorbic acid ethyl ether, etc., ascorbic acid glucosides such as ascorbic acid-2-glucoside, etc., and fatty acid esters thereof, ascorbic acid derivatives such as ascorbyl tocopheryl phosphate, etc.; vitamin derivatives of tocopherol derivatives, etc., such as tocopherol nicotinate, tocopherol acetate, tocopherol linoleate, tocopherol ferulate, tocopherolphosphoric acid ester, etc., tocotrienols, and other various kinds of vitamin derivatives as preferable materials.

As the hair-growth agents, blood circulation promoters and stimulants, there may be mentioned plant extracts and tinctures such as swertia herb extract, capsicum tincture, ginger tincture, ginger extract, cantharis tincture, etc.; capsaicin, nonanoic acid vanillylamide, zingerone, ichthammol, tannic acid, borneol, cyclandelate, cinnarizine, tolazoline, acetylcholine, verapamil, cepharanthine, γ-oryzanol, vitamin E and derivatives of nicotinic acid tocopherol-tocopherol acetate, etc., γ-oryzanol, nicotinic acid and nicotinic acid amide-nicotinic acid benzyl ester-inositol hexanicotinate, derivatives of nicotinyl alcohol, etc., allantoin, photosensitizer 301, photosensitizer 401, capronium chloride, pentadecanoic acid monoglyceride, flavanonol derivatives, stigmasterol or stigmastanol and its glycosides, and minoxidil as preferable materials.

As the hormones, there may be mentioned estradiol, estrone, ethinyl estradiol, cortisone, hydrocortisone, prednisone, etc., as preferable materials. As the other medicinal agents such as the anti-wrinkle agents, anti-aging agents, tightening agents, cooling sensation agents, thermal sensation agents, wound healing accelerators, irritation mitigating agents, analgesics, cell activators, etc., there may be mentioned retinols, retinoic acids, tocopheryl retinoate; lactic acid, glycolic acid, gluconic acid, fruit acid, salicylic acid and derivatives such as its glycosides and esterified products, etc., α- or β-hydroxy acids and its derivatives such as hydroxycapric acid, long-chain α-hydroxy fatty acid, long-chain α-hydroxy fatty acid cholesteryl, etc.; γ-aminobutyric acid, γ-amino-β-hydroxybutyric acid; carnitine; carnosine; creatine; ceramides, sphingosines; caffeine, xanthine, etc., and its derivatives; antioxidants and active oxygen scavengers such as coenzyme Q10, carotene, lycopene, astaxanthin, lutein, α-lipoic acid, platinum nanocolloids, fullerenes, etc.; catechins; flavones such as quercetin, etc.; isoflavones; gallic acid and ester sugar derivatives; tannin, sesamin, proanthocyanidin, chlorogenic acid, polyphenols such as apple polyphenol, etc.; rutin and derivatives such as glycosides, etc.; hesperidin and derivatives such as glycosides, etc.; lignan glycosides; licorice extract-related substances such as glabridin, glabrene, liquiritin, isoliquiritin, etc.; lactoferrin; shogaol, gingerol; fragrance substances and its derivatives such as menthol, cedrol, etc.; capsaicin, vanillin, etc., and derivatives; insect repellents such as diethyltoluamide, etc.; and complexes of physiologically active substances and cyclodextrins as preferable materials.

As the plant, animal and microbial extracts, there may be mentioned extracts such as iris extract, Angelica keiskei extract, hiba arborvitae extract, asparagus extract, avocado extract, sweet hydrangea leaf extract, almond extract, althea extract, arnica extract, aloe extract, apricot extract, apricot kernel extract, ginkgo biloba extract, Artemisia Capillaris Flower extract, fennel extract, turmeric extract, oolong tea extract, bearberry leaf extract, rose fruit extract, Echinacea angustifolia leaf extract, isodon japonica extract, scutellariae radix extract, cork tree bark extract, coptis rhizome extract, barley extract, Panax ginseng extract, Hypericum erectum extract, Lamium album extract, ononis spinosa extract, Nasturtium officinale extract, orange extract, seawater dried product, seaweed extract, persimmon leaf extract, Pyracantha Fortuneana Fruit extract, hydrolyzed elastin, hydrolyzed wheat powder, hydrolyzed silk, pueraria root extract, chamomilla extract, oil-soluble chamomilla extract, carrot extract, Artemisia capillaris extract, wild oats extract, Gentianella alborocea extract, licorice extract, oil-soluble licorice extract, kiwi fruit extract, kiou extract, Jew's Ear Fungus extract, cinchona extract, cucumber extract, paulownia leaf extract, guanosine, guava extract, Sophora root extract, gardenia extract, Sasa albo-marginata extract, Sophora angustifolia extract, walnut extract, chestnut extract, grapefruit extract, clematis extract, black rice extract, brown sugar extract, black vinegar, chlorella extract, mulberry extract, Gentiana lutea extract, Geranium thunbergii extract, black tea extract, yeast extract, magnolia bark extract, coffee extract, burdock extract, rice extract, rice fermented extract, rice bran fermented extract, rice germ oil, comfrey extract, collagen, cowberry extract, Asiasari radix extract, bupleuri radix extract, umbilical cord extract, saffron extract, salvia extract, common soapwort extract, sasa extract, hawthorn extract, sansha extract, Zanthoxylum piperitum extract, shiitake mushroom extract, Rehmannia glutinosa extract, Lithospermum root extract, perilla extract, Tilia japonica extract, Filipendula multijuga extract, Jatoba extract, poeny extract, ginger extract, Calamus extract, white birch extract, snow fungus extract, common horsetail extract, stevia extract, stevia fermented product, Chinese tamarisk extract, English ivy extract, Crataegus oxyacantha extract, Sambucus nigra extract, Achillea Millefolium extract, Mentha piperita extract, sage extract, common mallow extract, cnidium rhizome extract, swertia herb extract, mulberry bark extract, rhubarb extract, soybean extract, jujube extract, thyme extract, dandelion extract, lichen extract, tea extract, clove extract, cogongrass extract, citrus unshiu peel extract, tea tree oil, sweet tea extract, capsicum extract, Angelica acutiloba extract, Calendula officinalis extract, peach kernel extract, spruce extract, chameleon plant extract, tomato extract, fermented soybeans extract, carrot extract, garlic extract, bramble extract, hibiscus extract, ophiopogon tuber extract, lotus extract, parsley extract, birch extract, honey, hamamelis extract, parietaria extract, Isodon japonicus extract, bisabolol, Japanese cypress extract, Lactobacillus bifidus extract, loquat extract, coltsfoot extract, Petasites japonicus extract, Poria Sclerotium extract, btchers broom extract, grape extract, grape seed extract, propolis, loofah extract, safflower extract, peppermint extract, linden extract, Paeonia suffruticosa extract, hop extract, Romanas rose flower extract, pine tree extract, horse chestnut extract, skunk cabbage extract, washnut extract, lemon balm extract, mozuku extract, peach extract, cornflower extract, eucalyptus extract, saxifraga extract, Citrus junos extract, lily extract, coix seed extract, mugwort extract, lavender extract, green tea extract, eggshell membrane extract, apple extract, rooibos tea extract, litchi extract, lettuce extract, lemon extract, forsythia extract, astragalus extract, rose extract, rosemary extract, Anthemis nobilis extract, royal jelly extract, burnet extract, etc., as preferable materials.

As the antipruritic agents, there may be mentioned diphenhydramine hydrochloride, chlorpheniramine maleate, camphor, substance-P inhibitor, etc.

As the keratin exfoliating and dissolving agents, there may be mentioned salicylic acid, sulfur, resorcin, selenium sulfide, pyridoxine, etc.

As the antiperspirants, there may be mentioned chlorohydroxyaluminum, aluminum chloride, zinc oxide, zinc paraphenolsulfonate, etc.

As the algefacients, there may be mentioned menthol, methyl salicylate, etc.

As the astringents, there may be mentioned citric acid, tartaric acid, lactic acid, aluminum - potassium sulfate, tannic acid, etc.

As the enzymes, there may be mentioned superoxide dismutase, catalase, lysozyme chloride, lipase, papain, pancreatin, protease, etc.

As the nucleic acids, there may be mentioned ribonucleic acid and its salt, deoxyribonucleic acid and its salt, and adenosine triphosphate disodium as preferable materials.

As the fragrances, there may be mentioned synthetic fragrances and natural fragrances, and various kinds of mixed fragrances such as acetyl cedrene, amylcinnamaldehyde, allyl amyl glycolate, β-ionone, Iso E Super, isobutylquinoline, iris oil, irone, indole, ylang ylang oil, undecanal, undecenal, γ-undecalactone, estragole, eugenol, oakmoss, opoponax resinoid, orange oil, eugenol, auranthiol, galacsolid, carvacrol, L-carvone, camphor, canon, carrot seed oil, clove oil, methyl cinnamate, geraniol, geranyl nitrile, isobornyl acetate, geranyl acetate, dimethyl benzyl carbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinene, Triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexyl cinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methyl nonyl acetaldehyde, γ-methyl ionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, various essential oils, etc., as preferable materials.

As the pigments, coloring agents, dyes and pigments, there may be mentioned legal pigments such as brown No. 201, black No. 401, purple No. 201, purple No. 401, blue No. 1, blue No. 2, blue No. 201, blue No. 202, blue No. 203, blue No. 204, blue No. 205, blue No. 403, blue No. 404, green No. 201, green No. 202, green No. 204, green No. 205, green No. 3, green No. 401, green No. 402, red No. 102, red No. 104-1, red No. 105-1, red No. 106, red No. 2, red No. 201, red No. 202, red No. 203, red No. 204, red No. 205, red No. 206, red No. 207, red No. 208, red No. 213, red No. 214, red No. 215, red No. 218, red No. 219, red No. 220, red No. 221, red No. 223, red No. 225, red No. 226, red No. 227, red No. 228, red No. 230-1, red No. 230-2, red No. 231, red No. 232, red No. 3, red No. 401, red No. 404, red No. 405, red No. 501, red No. 502, red No. 503, red No. 504, red No. 505, red No. 506, orange No. 201, orange No. 203, orange No. 204, orange No. 205, orange No. 206, orange No. 207, orange No. 401, orange No. 402, orange No. 403, yellow No. 201, yellow No. 202-1, yellow No. 202-2, yellow No. 203, yellow No. 204, yellow No. 205, yellow No. 4, yellow No. 401, yellow No. 402, yellow No. 403-1, yellow No. 404, yellow No. 405, yellow No. 406, yellow No. 407, yellow No. 5, etc.; other acid dyes such as Acid Red 14, etc.; basic dyes such as Arianor Sienna Brown, Arianor Madder Red, Arianor Steel Blue, Arianor Straw Yellow, etc.; nitro dyes such as HC Yellow 2, HC Yellow 5, HC Red 3,4-hydoxy-propylamino-3-nitrophenol, N,N'-bis(2-hydroxyethyl)-2-nitro-p-phenylenediamine, HC Blue 2, Basic Blue 26, etc.; disperse dyes; inorganic white pigments such as titanium dioxide, zinc oxide, etc.; inorganic red pigments such as iron oxide (bengala), iron titanate, etc.; inorganic brown pigments such as $\gamma$-iron oxide, etc.; inorganic yellow pigments such as yellow iron oxide, ocher, etc.; inorganic black pigments such as black iron oxide, low-order titanium oxide, etc.; inorganic purple pigments such as mango violet, cobalt violet, etc.; inorganic green pigments such as chromium oxide, chromium hydroxide, cobalt titanate, etc.; inorganic blue pigments such as ultramarine, Prussian blue, etc.; pearl pigments such as titanium oxide coated mica, titanium oxide coated bismuth oxychloride, titanium oxide coated talc, colored titanium oxide coated mica, bismuth oxychloride, fish scale, etc.; metal powder pigments such as aluminum powder, copper powder, gold, etc.; surface treated inorganic and metal powder pigments; organic pigments such as zirconium, barium or aluminum lake, etc.; surface treated organic pigments; anthraquinones such as astaxanthin, alizarin, etc., naphthoquinones such as anthocyanidins, $\beta$-carotene, catenal, capsanthin, chalcone, carthamine, quercetin, crocin, chlorophyll, curcumin, cochineal, shikonin, etc., natural pigments and dyes such as bixin, flavones, betacyanidin, henna, hemoglobin, lycopene, riboflavin, rutin, etc.; oxidative dye intermediates and couplers such as p-phenylenediamine, toluene-2,5-diamine, o-, m-, or p-aminophenol, m-phenylenediamine, 5-amino-2-methylphenol, resorcine, 1-naphthol, 2,6-diaminopyridine, etc., and salts thereof, etc.; autoxidative dyes such as indoline, etc.; and dihydroxyacetone as preferable materials.

As the antiphlogistics and anti-inflammatory agents, there may be mentioned glycyrrhizic acid and its derivatives, glycyrrhetinic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphenhydramine hydrochloride, chlorpheniramine maleate; and plant extracts such as peach leaf extract, mugwort leaf extract, etc., as preferable materials.

As the anti-asthmatic agents, anti-chronic obstructive pulmonary disease agents, anti-allergic agents and immunomodulators, there may be mentioned aminophylline, theophyllines, steroids (fluticasone, beclomethasone, etc.), leukotriene antagonists, thromboxane inhibitors, intal, $\beta2$ stimulants (formoterol, salmeterol, albuterol, tulobuterol, clenbuterol, epinephrine, etc.), tiotropium, ipratropium, dextromethorphan, dimemorphan, bromhexine, tranilast, ketotifen, azelastine, cetirizine, chlorpheniramine, mequitazine, tacrolimus, cyclosporin, sirolimus, methotrexate, cytokine regulators, interferon, omalizumab, protein/antibody preparations as preferable materials.

As the anti-infective agents and antifungal agents, there may be mentioned oseltamivir, zanamivir and itraconazole as preferable materials. In addition to these, it is possible to add known cosmetic ingredients, pharmaceutical ingredients, food ingredients, etc., such as ingredients described in Japanese Standards of Cosmetic Ingredients, Japanese Standard of Cosmetic Classification Formulating Components, Japan Cosmetic Industry Association ingredient display name list, INCI Dictionary (The International Cosmetic Ingredient Dictionary and Handbook), Japanese Standards of Quasi-drug Ingredients, Japanese Pharmacopoeia, Japanese Pharmaceutical Excipients, Japan's Specifications and Standards for Food Additives, etc., and, ingredients described in patent gazettes and patent gazettes of unexamined patent applications (including publication gazettes and republications) of Japan and foreign countries whose International Patent Classification IPC belong to the classifications of A61K7 and A61K8, etc., in known combinations and mixing ratios and amounts.

The particles of the present invention are excellent in light scattering properties, so that they can be applied to the industrial field as a light diffusing agent, etc. The particles of the present invention may be utilized itself or in a form in which they are dispersed with an optional additive(s) in an appropriate medium(s) depending on their uses, as a light diffusing agent or a light diffusing composition. For example, they are expected to be utilized as a light diffusing agent or a light diffusing composition of a diffusion sheet for lighting or a backlight, or a window film or a decorative sheet for building materials.

EXAMPLES

[Example 1: Bamboo-derived cellulose particles]

After adding 1343 g of pure water to 407 g of bamboo-derived cellulose nanofiber having a solid content of 10% by mass (manufactured by Chuetsu Pulp & Paper Co., Ltd.), the mixture was stirred by a mechanical stirrer for 1 hour to disperse the cellulose nanofiber. This dispersion liquid was pulverized by a wet-type pulverization apparatus Star Brust (manufactured by SUGINO MACHINE LIMITED) at a pressure of 220 MPa 50 times to obtain a dispersion liquid of the bamboo-derived cellulose nanofiber. The obtained dispersion liquid was weighed and 10 g thereof was charged in a petri dish, and dried at 105°C for 1 hour to remove water content. An amount of the obtained residue was measured, and a cellulose concentration (solid content concentration) in water was calculated from the weight difference before and after drying. As a result, the concentration was 2.0% by mass.

1,575 g of the above-mentioned dispersion liquid of the bamboo-derived cellulose nanofiber was spray-dried for 75 minutes at an atomizer rotation speed of 30,000 rpm, an inlet temperature of 180°C, and a cyclone differential pressure of 0.5 kPa to obtain 22.6 g of powder as the title particles. During this time, the outlet temperature changed between 130°C and 117°C.

[Example 2: Broadleaf tree-derived cellulose particles]

After adding 400 g of pure water to 400 g of broadleaf tree-derived cellulose nanofiber (manufactured by Daio Paper Corporation) having a solid content of 2% by mass, the mixture was stirred by a mechanical stirrer for 1 hour to disperse the broadleaf tree-derived cellulose nanofiber. 800 g of this dispersion liquid was spray-dried for 26 minutes at an atomizer rotation speed of 30,000 rpm, an inlet temperature of 182°C, and a cyclone differential pressure of 0.5 kPa to obtain 1.01 g of powder as the title particles. During this time, the outlet temperature changed between 102°C and 77°C.

[Example 3: Acicular tree-derived cellulose particles]

After adding 400 g of pure water to 400 g of acicular tree-derived cellulose nanofiber (manufactured by Daio Paper Corporation) having a solid content of 2% by mass, the mixture was stirred by a mechanical stirrer for 1 hour to disperse the acicular tree-derived cellulose nanofiber. 800 g of this dispersion liquid was spray-dried for 26 minutes at an atomizer rotation speed of 30,000 rpm, an inlet temperature of 178°C, and a cyclone differential pressure of 0.5 kPa to obtain 2.70 g of powder as the title particles. During this time, the outlet temperature was constant at 17°C.

[Example 4: Crystalline cellulose particles]

75 g of crystalline cellulose (Fushimi Pharmaceutical Co., Ltd., Comprecel M101) was dispersed in 750 g of pure water. This dispersion liquid was pulverized by a wet-type pulverization apparatus Star Brust (manufactured by SUGINO MACHINE LIMITED Co., Ltd.) at a pressure of 220 MPa 30 times to obtain a dispersion liquid of the crystalline cellulose. The obtained dispersion liquid was weighed and 10 g thereof was charged in a petri dish, and dried at 105°C for 1 hour to remove water content. An amount of the obtained residue was measured, and a cellulose concentration (solid content concentration) in water was calculated from the weight difference before and after drying. As a result, the concentration was 4.0% by mass.

250 g of pure water was added to 250 g of the above-mentioned dispersion liquid to prepare 2.0% by mass of the dispersion liquid of the crystalline cellulose, which was spray-dried for 23 minutes at an atomizer rotation speed of 20,000 rpm, an inlet temperature of 160°C, and a cyclone differential pressure of 0.5 kPa to obtain 4.6 g of powder as the title particles. During this time, the outlet temperature changed between 109°C and 95°C.

[Example 5: Crystalline cellulose particles]

500 g of 4.0% by mass dispersion liquid of the crystalline cellulose obtained in Example 4 was spray-dried for 25 minutes at an atomizer rotation speed of 20,000 rpm, an inlet temperature of 160°C, and a cyclone differential pressure of 0.5 kPa to obtain 12.1 g of powder as the title particles. During this time, the outlet temperature changed between 109°C and 93°C.

[Evaluation Example 1: Measurement of distribution of reflected light]

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, NICETACK (Registered Trademark) NW-10S was pasted on the above-mentioned black drawing paper, and after pressing the sample powder thereon, excess powder was removed by an air gun adjusted to a pressure of 0.2 MPa. Measurement of the distribution of the reflected light was carried out by a goniophotometer GP-5 (manufactured by Murakami Color Research Laboratory Co., Ltd.). The measurement incident light was carried out at three points of -30, -45 and -60°. The light scattering rate at each incident light angle was calculated in accordance with the following formula (1) (see WO 2010/092890).

$$\text{Light scattering rate (\%)} = \frac{(\text{Reflection intensity at angle of } 20°/\cos 20°) + (\text{Reflection intensity at angle of } 70°/\cos 70°)}{2 \times (\text{Reflection intensity at angle of } 5°/\cos 5°)} \times 100$$

$$\cdots \ (1)$$

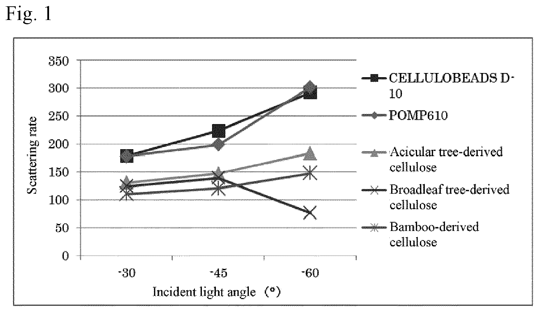

The samples used were bamboo-, broadleaf tree- and acicular tree-derived cellulose particles obtained in Examples 1 to 3. Also, as Comparative Examples 1 and 2, commercially available cellulose particles CELLULOBEADS D-10 (manufactured by Daito Kasei Kogyo Co., Ltd.) and porous Nylon 6 (polyamide 6) powder POMP610 (Registered Trademark) (manufactured by Toabo Corporation). The reflection intensity and the light scattering rate of the samples for each incident light are shown in Tables 1 to 3. In addition, the correlation between the incident light angle and the light scattering rate of each sample is shown in Table 4 and FIG. 1. Further, graphs showing the reflection intensities of the samples of Example 1 and Comparative Example 1 at the incident light of -45° are shown in FIG. 2(a) and (b).

[Table 1]

| Results at incident light of -30° | | Reflection intensity at angle of 5° | Reflection intensity at angle of 20° | Reflection intensity at angle of 70° | Scattering rate (%) |
|---|---|---|---|---|---|
| Evaluated sample | | | | | |
| Example 1 | Bamboo-derived cellulose | 50.72 | 48.23 | 20.92 | 110.47 |
| Example 2 | Broadleaf tree-derived cellulose | 40.87 | 40.57 | 20.11 | 124.28 |
| Example 3 | Acicular tree-derived cellulose | 40.82 | 41.06 | 21.65 | 130.56 |
| Comparative Example 1 | CELLULOBEADS D-10 | 21.51 | 28.34 | 16.08 | 178.71 |
| Comparative Example 2 | POMP610 | 21.52 | 28.16 | 16.03 | 177.84 |

[Table 2]

| Results at incident light of -45° | | Reflection intensity at angle of 5° | Reflection intensity at angle of 20° | Reflection intensity at angle of 70° | Scattering rate (%) |
|---|---|---|---|---|---|
| Evaluated sample | | | | | |
| Example 1 | Bamboo-derived cellulose | 48.83 | 46.92 | 23.34 | 120.54 |
| Example 2 | Broadleaf tree-derived cellulose | 41.03 | 41.48 | 24.17 | 139.38 |
| Example 3 | Acicular tree-derived cellulose | 41.29 | 42.4 | 26.35 | 147.37 |
| Comparative Example 1 | CELLULOBEADS D-10 | 22.07 | 29.06 | 23.33 | 223.74 |
| Comparative Example 2 | POMP610 | 23.58 | 28.36 | 21.89 | 198.95 |

[Table 3]

| Results at incident light of -60° | | | | | |
|---|---|---|---|---|---|
| Evaluated sample | | Reflection intensity at angle of 5° | Reflection intensity at angle of 20° | Reflection intensity at angle of 70° | Scattering rate (%) |
| Example 1 | Bamboo-derived cellulose | 42.94 | 42.22 | 28.18 | 147.69 |
| Example 2 | Broadleaf tree-derived cellulose | 41.42 | 40.73 | 6.97 | 76.63 |
| Example 3 | Acicular tree-derived cellulose | 38.6 | 41.01 | 33.63 | 183.20 |
| Comparative Example 1 | CELLULOBEADS D-10 | 21.17 | 28.53 | 32.16 | 292.67 |
| Comparative Example 2 | POMP610 | 21.45 | 28.33 | 34.08 | 301.39 |

[Table 4]

| Relationship between incident light angle and scattering rate | | | | |
|---|---|---|---|---|
| Evaluated sample | | Scattering rate at incident light of -30° | Scattering rate at incident light of -45° | Scattering rate at incident light of -60° |
| Example 1 | Bamboo-derived cellulose | 110.47 | 120.54 | 147.69 |
| Example 2 | Broadleaf tree-derived cellulose | 124.28 | 139.38 | 76.63 |
| Example 3 | Acicular tree-derived cellulose | 130.56 | 147.37 | 183.20 |
| Comparative Example 1 | CELLULOBEADS D-10 | 178.71 | 223.74 | 292.67 |
| Comparative Example 2 | POMP610 | 177.84 | 198.95 | 301.39 |

[Evaluation Example 2: Observation of morphology of particles]

The bamboo-, broadleaf tree- and acicular tree-derived cellulose particles obtained in Examples 1 to 3 and the crystalline cellulose particles obtained in Examples 4 and 5 were used as samples. Each sample was pasted to a carbon tape, and morphological observation was carried out by using a scanning electron microscope Miniscope (Registered Trademark) TM3000 (manufactured by Hitachi High-Tech Corporation). The results of morphological observation of each cellulose particle are shown in FIGS. 3(a) to (e), respectively.

[Evaluation Example 3: Cross-section observation]

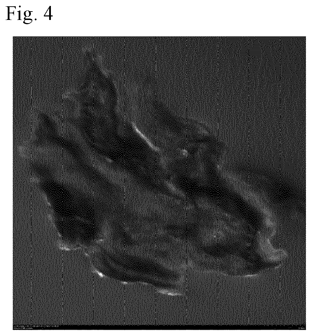

The bamboo-derived cellulose particles of Example 1 were embedded in a thermosetting resin mixed solution comprising 50 mL of Epok 812 as a main agent, 44.5 mL of Methyl Nadic Anhydride as a curing agent, and 1.3 mL of Tri-Dimethyl Aminomethyl Phenol as a polymerization accelerator, and the mixture was heated at 60°C for 12 hours to cure the same. A thin piece with a thickness of 150 nm was prepared by an ultramicrotome (manufactured by Leica Microsystems) from the obtained cured product, and the thin pieced sample was sampled on a sheet mesh and observed by a transmission type electron microscope. The results are shown in FIG. 4.

[Evaluation Example 4: Measurement of hardness]

Hardnesses of the bamboo-derived cellulose particles obtained in Example 1, and commercially available cellulose particles CELLULOBEADS D-10 (manufactured by Daito Kasei Kogyo Co., Ltd.) of Comparative Example 1 were measured by ENT-2100 (manufactured by ELIONIX INC.). Using φ 50 μm flat indenter, a set load was set to 20 mN, and a loading time was set to 10 seconds. Each sample was spread on a silicon substrate, and after measuring each 5 particles, an average value of the obtained measured values was calculated. As a result, the hardness of the bamboo-derived cellulose particles was 23.75 MPa, and the hardness of CELLULOBEADS D-10 was 42.91 MPa.

[Evaluation Example 5: Evaluation of average friction coefficient and fluctuation of average friction coefficient]

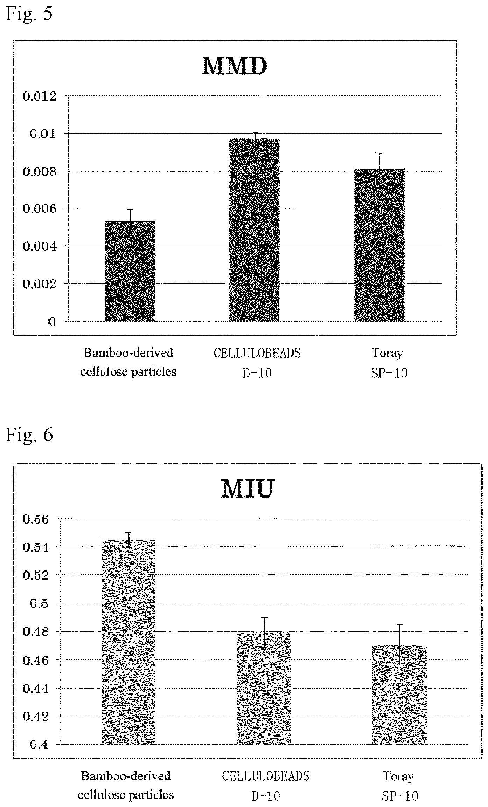

0.3 g of each sample was weighed, and a friction coefficient and fluctuation of the friction coefficient were evaluated by a friction tester KES-SE (manufactured by Kato Tech Co., Ltd.) equipped with a 10 mm square silicon material sensor. Measurements of each sample were carried out 5 times. As for the samples, the bamboo-derived cellulose particles obtained in Example 1, and CELLULOBEADS D-10 (manufactured by Daito Kasei Kogyo Co., Ltd.) and Toray nylon fine particles SP-10 (manufactured by Toray Industries, Inc.) as controls were used. Incidentally, as the sensor, a 10 mm square silicon wire was used, and a measurement distance was set to 20 mm, a static load was set to 25 gf, a measurement speed was set to 1.0 mm/sec, and a contact surface width was set to 10 mm. The results are shown in FIG. 5 and FIG. 6.

In FIG. 5, MMD refers to the fluctuation of the average friction coefficient, and is an index that represents the feeling of smoothness and roughness that is felt when a person's finger touches the surface of an object. When the numerical value of MMD is smaller, it is smoother.

In FIG. 6, MIU refers to the average friction coefficient, and is an index that represents the degree of slipperiness or slipperiness that is felt when a person's finger touches the surface of an object. When the numerical value of MIU is smaller, it is easier to slip.

[Evaluation Example 6: Powder X-ray diffraction measurement of cellulose particles and calculation of crystallization degree]

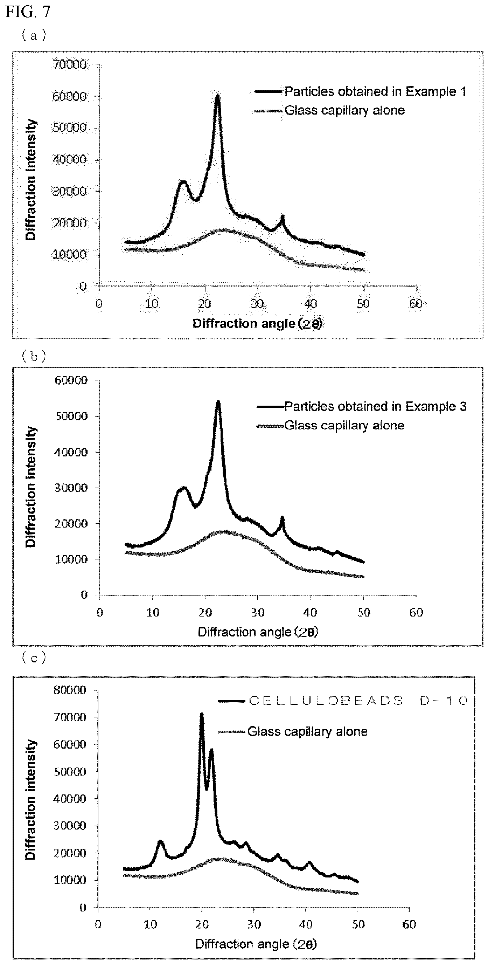

Each sample powder was enclosed in a Lindeman glass capillary (φ 0.7 mm), and powder X-ray diffraction measurement was carried out by using X'Pert PRO MPD (manufactured by PANalytical). Cu/Kα-radiation was used as an X-ray source, and measurement was carried out with tube voltage of 45 kV, tube current of 40 mA, measurement range of diffraction angle $2\theta$ = 5 to 50°, step width of 0.05°, and average time/STEP of 500 seconds/STEP. As samples, cellulose particles obtained in Examples 1 and 3, and commercially available cellulose particles CELLULOBEADS D-10 of Comparative Example 1 were used. The respective X-ray diffraction patterns are shown in FIG. 7(a) to (c).

From FIG. 7(a) and (b), it was found that the crystal forms of the cellulose particles obtained in Examples 1 and 3 were the same (hereinafter, referred to as type I crystal form). On the other hand, from comparison of FIG. 7(a) and (b) with FIG. 7(c), it was found that the crystal form of CELLULOBEADS D-10 was different from those of the cellulose particles obtained in Example 1 or 3 (hereinafter, referred to as type II crystal form).

Next, the crystallization degree of each particle was calculated from the obtained powder X-ray diffraction patterns. For calculation of the crystallization degree, the following equations (2) and (3) were used (see SEN-I GAKKAISHI, Vol. 46, No. 8 (1990), 324).

$$\text{Crystallization degree of cellulose type I crystal form (\%)} = \{ (I_{22.6} - I_{22.6,blank}) - (I_{18.5} - I_{18.5,blank}) \} / (I_{22.6} - I_{22.6,blank}) \times 100 \quad \cdots (2)$$

(wherein $I_{22.6}$ and $I_{18.5}$ indicate diffraction intensities at diffraction angles $2\theta$ of 22.6° and 18.5°, and $I_{22.6,blank}$ and $I_{18.5,blank}$ each indicate diffraction intensities of only the glass capillaries at diffraction angles $2\theta$ of 22.6° and 18.5°.)

$$\text{Crystallization degree of cellulose type II crystal form (\%)} = \{ (I_{20.0} - I_{20.0,blank}) - (I_{15.0} - I_{15.0,blank}) \} / (I_{20.0} - I_{20.0,blank}) \times 100 \quad \cdots (3)$$

(wherein $I_{20.0}$ and $I_{15.0}$ indicate diffraction intensities at diffraction angles $2\theta$ of 20.0° and 15.0°, and $I_{20.0,blank}$ and $I_{15.0,blank}$ each indicate diffraction intensities of only the glass capillaries at diffraction angles $2\theta$ of 20.0° and 15.0°.)

As a result of calculation, the crystallization degrees of the cellulose particles obtained in Examples 1 and 3 were both 70%. On the other hand, the crystallization degree of the commercially available cellulose particles CELLULOBEADS D-10 of Comparative Example 1 was 90%.

[Evaluation Example 7: Particle size evaluation of cellulose particles]

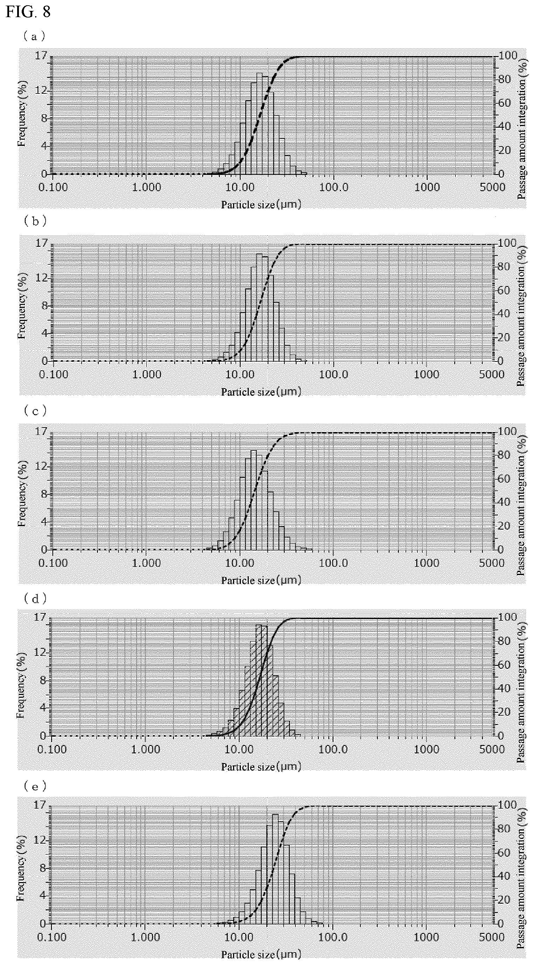

Using a scattering type particle size distribution measuring device LA-960 (manufactured by Horiba, Ltd.), various kinds of the sample concentrations were adjusted so that the transmittance of a semiconductor laser (650 nm) and a light emitting diode (405 nm) became 90.0% or less with the state that the sample solution was circulated with a circulation rate of 3 and a stirring rate of 2. After the concentration was adjusted, ultrasonic waves were irradiated at an ultrasonic wave intensity 3 for 1 minute, and then the particle size measurement was carried out. The results are shown in FIGS. 8(a) to (e).

[Evaluation Example 8: Measurement of distribution of reflected light in foundation prescription]

In accordance with Table 5, A phase and B phase were each weighed into two 300 mL tall beakers, and each heated and dissolved at about 75°C. A phase was added to the above-mentioned heated B phase, and QUICK HOMO MIXER (manufactured by Mizuho Industrial Co., Ltd., 5,000 rpm, 3 minutes) treatment was carried out. Thereafter, the mixture was stirred and cooled with a LABORATORY HIGH POWER MIXER (manufactured by AS ONE Corporation) until the liquid temperature reached about 35°C. In a microtube STF-15 (manufactured by SANSHO Co., Ltd.) having a volume of 1.5 mL were each weighed 450 mg of the prepared water in oil (hereinafter abbreviated to as w/o) foundation liquid and 50 mg of the evaluation particle powder, and the mixture was uniformly mixed and kneaded with a dispensing spoon to prepare w/o foundation samples each containing 5% of the evaluation particles. As the evaluation particles, cellulose particles obtained in Examples 1 to 3 were used. Also, as Comparative Example, commercially available cellulose particles CELLULOBEADS D-10 (manufactured by Daito Kasei Kogyo Co., Ltd.) were used.

[Table 5]

| Phase | Component | Composition |
|---|---|---|
| A | Nikkomulese WO-CF PLUS[*1] | 5.000% |
| | SILBLEND-91[*1] | 24.000% |
| | KF-96L-2CS[*2] | 3.000% |
| | KF6028[*2] | 1.500% |
| | DISM[*1] | 2.000% |
| | FAS70CSI-E[*1] | 5.000% |
| | Fresh color base[*1] | 10.000% |
| B | conc. Glycerin[*3] | 8.000% |
| | 1,3-BG[*4] | 3.000% |
| | Sodium chloride[*5] | 0.500% |
| | EDTA-2Na[*6] | 0.050% |
| | Ethanol | 3.000% |
| | Methyl 4-Hydroxybenzoate[*7] | 0.050% |
| | Purified water | 34.900% |
| Total | | 100.000% |

*1: manufactured by Nikko Chemicals Co., Ltd.
*2: manufactured by Shin-Etsu Chemical Co., Ltd.
*3: manufactured by Sakamoto Yakuhin Kogyo Co., Ltd.
*4: manufactured by NH Neochem Co., Ltd.
*5: manufactured by FUJIFILM Wako Pure Chemical Corporation
*6: manufactured by Junsei Chemical Co., Ltd.
*7: manufactured by Tokyo Chemical Industry Co., Ltd.

Nikkomulese WO-CF PLUS: PEG-10 dimethicone, dimethicone, disteardimonium hectorite, lauryl PEG-9 polydimethylsiloxyethyl dimethicone, tocopherol
SILBLEND-91: Cyclopentasiloxane, dimethicone, (dimethicone/vinyldimethicone) crosspolymer
KF-96L-2CS: Dimethicone
KF6028: PEG-9 polydimethylsiloxyethyl dimethicone
DISM: Diisostearyl malate
FAS70CSI-E: Titanium oxide dispersion product (titanium oxide, cyclopentasiloxane, PEG/PPG-18/18 dimethicone, triethoxycaprylsilane, alumina, tocopherol acetate, silica) Fresh color base: titanium oxide, cyclopentasiloxane, PEG/PPG-18/18 dimethicone, iron oxide, methicone, tocopherol acetate
1,3-BG: 1,3-Butylene glycol
EDTA-2Na: Ethylenediaminetetraacetic acid disodium salt

A black drawing paper PI-N86D (manufactured by Maruai Inc.) was pasted onto a micro slide glass S1111 (manufactured by Matsunami Glass Ind., Ltd.) using NICETACK (Registered Trademark) NW-10S (manufactured by Nichiban Co., Ltd.). Then, a w/o foundation containing 5% of each evaluation particles prepared above was spread uniformly on the above-mentioned black drawing paper and air-dried for 20 minutes. Measurement of the distribution of the reflected light was carried out by a goniophotometer GP-5 (manufactured by Murakami Color Research Laboratory Co., Ltd.). The measurement incident light was made -45°, and the light scattering rate was calculated in accordance with the formula (1) shown in Evaluation Example 1. Also, the reflection intensity at 45° was measured. The results are shown in Table 6.

[Table 6]

| Light scattering rate at incident light of -45° and reflection intensity at 45° | | | | | |
|---|---|---|---|---|---|
| Sample name | Reflection intensity at angle of 5° | Reflection intensity at angle of 20° | Reflection intensity at angle of 70° | Scattering rate (%) | Reflection intensity at angle of 45° |
| w/o foundation alone | 51.56 | 59.14 | 57.74 | 223.89 | 82.40 |
| w/o foundation containing 5% of Example 1 | 50.17 | 50.19 | 35.36 | 155.67 | 49.99 |
| w/o foundation containing 5% of Example 2 | 51.13 | 51.36 | 33.11 | 147.55 | 49.62 |
| w/o foundation containing 5% of Example 3 | 51.27 | 52.18 | 36.42 | 157.40 | 52.23 |
| w/o foundation containing 5% of CELLULOBEADS D-10 | 51.74 | 55.77 | 47.91 | 191.99 | 63.47 |

[Evaluation Example 9: Evaluation of oil absorption amount]

Evaluation of oil absorption was carried out with reference to the JIS standard (K 5101-13-1: 2004 (ISO 787-5: 1980)). To the evaluation particles powder weighed on a weighing dish, and the operation that linseed oil (manufactured by Summit Oil Mill Co., Ltd.) was gradually dropped with a spuit and the mixture was mixed and kneaded with a spatula each time was repeated, which dropping was continued until lumps of the linseed oil and the evaluation particles formed. The time at which the powder became smooth paste was made the end point. This paste should be such that it can be spread without cracking or shattering and that it lightly adheres to the measurement plate. From the evaluation particle powder weight (g) at the end point to the linseed oil dropped amount (g), the oil absorption amount (g/100 g) was calculated. As the evaluation particles, cellulose particles obtained in Examples 1 to 3 were used. Also, as Comparative Examples, commercially available cellulose particles CELLULOBEADS D-10 (manufactured by Daito Kasei Kogyo Co., Ltd.) and Celluflow C-25 (manufactured by JNC Corporation) were used. The results are shown in Table 7.

[Table 7]

| Oil absorption amount of evaluation particles | |
|---|---|
| Sample name | Oil absorption amount (g/100 g) |
| Example 1 | 62.8 |

(continued)

| Oil absorption amount of evaluation particles | |
|---|---|
| Sample name | Oil absorption amount (g/100 g) |
| Example 2 | 80.9 |
| Example 3 | 80.0 |
| CELLULOBEADS D-10 | 44.5 |
| Celluflow C-25 | 58.4 |

UTILIZABILITY IN INDUSTRY

The particles of the present invention are natural material and soft, and are excellent in light scattering properties. Accordingly, it is possible to apply them to an industrial field as a light diffusing agent, etc. In addition, the particles of the present invention are appropriately suppressed in the crystallization degree, have a wrinkle-like or fold-like uneven structure on the surface thereof so that they are soft, and have excellent optical characteristics (light scattering properties) that the incident light is uniformly light scattered so that they can be expected to exhibit a defocus effect (also referred to as a soft focus effect), whereby it is preferable to apply them to a cosmetic field that comes into direct contact with the skin. In particular, the particles of the present invention are also excellent in oil absorption properties, so that it is suitable for adding them to cosmetics containing an oily component(s) and an oily base(s).

**Claims**

1.   A particle containing cellulose or a cellulose derivative as a main component, which comprises having a wrinkle-like or fold-like uneven structure on a surface and a crystallization degree of 80% or less.

2.   The particle containing cellulose or a cellulose derivative as a main component, which comprises having a light scattering rate in a range of 50 to 230%.

3.   The particle according to claim 1, wherein a light scattering rate is in a range of 50 to 230%.

4.   The particle according to claim 2, wherein a crystallization degree is 80% or less.

5.   The particle according to any one of claims 1 to 4, wherein the cellulose is plant-derived cellulose, cellulose fiber or cellulose nanofiber.

6.   A method of producing a particle containing cellulose or a cellulose derivative as a main component, having a wrinkle-like or fold-like uneven structure on a surface and a crystallization degree of 80% or less, which comprises a step of obtaining a dispersion liquid of the cellulose or the cellulose derivative, and a step of spray-drying the obtained dispersion liquid.

7.   A method of producing a particle containing cellulose or a cellulose derivative as a main component and having a light scattering rate in a range of 50 to 230%, which comprises a step of obtaining a dispersion liquid of the cellulose or the cellulose derivative, and a step of spray-drying the obtained dispersion liquid.

8.   The producing method according to claim 6, wherein a light scattering rate of the particle containing cellulose or a cellulose derivative as a main component is in a range of 50 to 230%.

9.   The producing method according to claim 7, wherein a crystallization degree of the particle containing cellulose or a cellulose derivative as a main component is 80% or less.

10.  The producing method according to any one of claims 6 to 9, wherein the cellulose is plant-derived cellulose, cellulose fiber or cellulose nanofiber.

**11.** The producing method according to any one of claims 6 to 10, wherein a concentration of the cellulose or the cellulose derivative in the dispersion liquid is 0.5 to 5% by mass.

**12.** The producing method according to any one of claims 6 to 11, wherein the dispersion liquid is obtained by physical pulverization of the cellulose or the cellulose derivative.

**13.** A cosmetic which comprises the particle according to any of claims 1 to 5, or the particle obtained by the producing method according to any of claims 6 to 12.

Fig. 1

Fig. 2

( a )

( b )

Fig. 3

(a)

(b)

(c)

(d)

(e)

Fig. 4

Fig. 5

Fig. 6

FIG. 7

(a)

(b)

(c)

FIG. 8

## INTERNATIONAL SEARCH REPORT

| International application No. |
| --- |
| PCT/JP2019/003646 |

**A. CLASSIFICATION OF SUBJECT MATTER**

Int.Cl.   C08B15/00(2006.01)i,   A61K8/73(2006.01)i,   A61Q1/00(2006.01)i,
A61Q19/00(2006.01)i

According to International Patent Classification (IPC) or to both national classification and IPC

**B. FIELDS SEARCHED**

Minimum documentation searched (classification system followed by classification symbols)
Int.Cl. C08E, A61K, A61Q, D21H

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y<br>A | 上谷幸治郎ら，液滴乾燥法によるナノセルロースの構造制御，セルロース学会第19回年次大会講演要旨集，01 July 2012, p. 108, non-official translation (UETANI, Kojiro et al., "Structural control of nanocellulose by droplet drying method", Lecture abstracts of the 19th Annual Meeting of the Cellulose Society of Japan) | 1-10, 12<br>13<br>11 |
| X<br>Y | JP 2017-78145 A (DAIO PAPER CORP.) 27 April 2017, paragraphs [0020], [0058], [0062], [0065]-[0068] (Family: none) | 1-12<br>13 |
| X<br>Y | JP2014-118521A (KAO CORP.) 30 June 2014, paragraphs [0048]-[0049], [0051] (Family: none) | 1-12<br>13 |

☒ Further documents are listed in the continuation of Box C.    ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 March 2019 (06.03.2019) | 26 March 2019 (26.03.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
|---|
| PCT/JP2019/003646 |

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | UETANI, Kojiro et al., Soft Matter, 2013, vol. 9, pp. 3396-3401 & Supporting Information | 13<br>1-12 |
| A | 中坪文明, セルロースナノファイバーの化学変成戦略, Nanocellulose Symposium 2013 「生物が創り出すナノ繊維」〜セルロースナノファイバー 広がる用途開発〜 要旨, 27 February 2013, pp. 1-6, URL:http://www.rish.kyoto-u.ac.jp/labm/wp-content/uploads/2013/04/cellulosesymposium2013.pdf, non-official translation (NAKATSUBO, Fumiaki, Chemical alteration strategy of cellulose nanofibers, "Nanofibers produced by organisms", – Cellulose nanofibers, Development of expanding application – summary") | 1-13 |
| A | 磯貝明, セルロースの高次構造と機能, 繊維機械学会誌, 1997, vol. 50, no. 4, pp. 181-185, (ISOGAI, Akira, "Cellulose : It's Higher-order Structures and Functionalities", Sen'i Kikai Gakkaishi (Journal of the Textile Machinery Society of Japan) | 1-13 |
| A | 磯貝明, TEMPO 酸化セルロースシングルナノファイバー複合材料, 日本ゴム協会誌, 2012, vol. 85, no. 12, pp. 388-393, (ISOGAI, Akira, "Composite Materials of TEMPO-Oxidized Cellulose Single Nanofiber", NIPPON GOMU KYOKAISHI) | 1-13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016040264 A
- WO 2010092890 A
- WO 2017056908 A
- JP HEI9132601 A

**Non-patent literature cited in the description**

- *SEN-I GAKKAISHI,* 1990, vol. 46 (8), 324